(19) Europäisches Patentamt
European Patent Office
Office européen des brevets

(11) EP 4 473 954 A1

(12) EUROPEAN PATENT APPLICATION

(43) Date of publication:
**11.12.2024 Bulletin 2024/50**

(21) Application number: **24179491.6**

(22) Date of filing: **03.06.2024**

(51) International Patent Classification (IPC):
***A61K 6/16*** *(2020.01)* ***A61K 6/20*** *(2020.01)*
***A61K 6/30*** *(2020.01)* ***A61K 6/60*** *(2020.01)*
***A61K 6/62*** *(2020.01)* ***A61K 6/887*** *(2020.01)*

(52) Cooperative Patent Classification (CPC):
(C-Sets available)
**A61K 6/887; A61K 6/16; A61K 6/20; A61K 6/30; A61K 6/60; A61K 6/62** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.06.2023 JP 2023092012**

(71) Applicant: **Shofu Inc.**
**Kyoto-shi, Kyoto 605-0983 (JP)**

(72) Inventors:
- **MIYATA, Shunske**
  **Kyoto, 605-0983 (JP)**
- **MATSUMOTO, Akiko**
  **Kyoto, 605-0983 (JP)**
- **YAMAMOTO, Kenzo**
  **Kyoto, 605-0983 (JP)**

(74) Representative: **Eisenführ Speiser**
**Patentanwälte Rechtsanwälte PartGmbB**
**Gollierstraße 4**
**80339 München (DE)**

# (54) DENTAL CURABLE COMPOSITION CONTAINING ULTRAVIOLET ABSORBER

(57) To provide a dental curable composition having excellent solubility of an ultraviolet absorber, sunlight stability, fluorescence property, and mechanical property.

To provide a dental curable composition comprising, (A) polymerizable monomer, (B) polymerization initiator, (C) fluorescent agent, and (D) ultraviolet absorber, wherein, the dental curable composition comprises, as the (D) ultraviolet absorber, (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the range of 1 to 4.

EP 4 473 954 A1

(52) Cooperative Patent Classification (CPC): (Cont.)

C-Sets
**A61K 6/20, C08L 33/08;**
**A61K 6/20, C08L 33/10;**
**A61K 6/30, C08L 33/08;**
**A61K 6/30, C08L 33/10;**
**A61K 6/887, C08L 33/08;**
**A61K 6/887, C08L 33/10**

## Description

BACKGROUND OF THE INVENTION

Field of the Invention

The present invention relates to a dental curable composition.

Description of the Related Art

A dental curable composition has been used for treatment of an oral cavity in the dental field, and applied to a dental adhesive material, a dental composite resin, a dental core build-up material, a dental resin cement, a dental coating material, a dental sealant material, a dental manicure material, a dental splinting material, a dental glass ionomer cement, a dental hard resin, a dental cutting and machining material, a dental 3D printer material, a dental orthodontic material and the like.
Patent Document 1 discloses a photopolymerizable composition containing an α-diketone and an aromatic tertiary amine as a photopolymerization initiator, and a benzotriazole compound as an ultraviolet absorber.
Patent Document 2 discloses a photopolymerizable composition containing a visible light polymerization initiator, a phthalate ester-based fluorescent agent, and a benzotriazole compound as an ultraviolet absorber.
Patent Document 3 discloses a dental photopolymerizable composition containing a photopolymerization initiator and two types of ultraviolet absorbers having absorption maximum in specific wavelength regions.
Patent Document 4 discloses a dental composition containing a fluorescent agent and an ultraviolet absorber that does not have an absorption maximum in a specific wavelength range.

Relevant References

Patent Literature

[Patent document 1] Japanese Unexamined Patent Application Publication No. 2004-231913
[Patent document 2] Japanese Unexamined Patent Application Publication No. 2014-15408
[Patent document 3] Japanese Unexamined Patent Application Publication No. 2016-166138
[Patent document 4] International Publication WO2020/066099

SUMMARY OF THE INVENTION

Technical Problem

However, in the dental curable compositions using a ultraviolet absorber described in Patent Documents 1 to 4, there has been a problem in solubility of an ultraviolet absorber with respect to the polymerizable monomer, sunlight stability, fluorescence, and mechanical property.
An object of the present invention is to provide a dental curable composition having excellent solubility of an ultraviolet absorber, sunlight stability, fluorescence, and mechanical strength.

Solution to Problem

The present invention provides a dental curable composition comprising, (A) polymerizable monomer, (B) polymerization initiator, (C) fluorescent agent, and (D) ultraviolet absorber, wherein,
the dental curable composition comprises, as the (D) ultraviolet absorber, (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4.

Advantageous Effects of Invention

The present invention can provide a dental curable composition having excellent solubility of an ultraviolet absorber, sunlight stability, fluorescence, and mechanical strength.

DETAILED DESCRIPTION OF THE PREFERRED EMBODIMENTS

In the present invention, the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4 may be a benzophenone compound represented by formula (1).

[Formula (1)]

[Chemical formula 1]

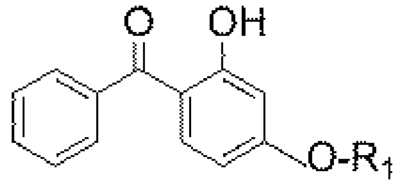

(In the formula (1), $R_1$ is an organic group having 2 to 10 carbon atoms.)

In the present invention, the dental curable composition may comprise a combination of (B-1) photosensitizer, (B-2) photoacid generator and (B-3) polymerization accelerator as the (B) polymerization initiator, the dental curable composition may comprise a diaryl iodonium salt compound as the (B-2) photoacid generator, and the dental curable composition may comprise a tertiary amine compound as the (B-3) polymerization accelerator.

In the present invention, the dental curable composition may not substantially comprise an aromatic amine compound as the (B-3) polymerization accelerator.

In the present invention, the dental curable composition may comprise only the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4 as the (D) ultraviolet absorber.

In the present invention, the dental curable composition may comprise only one type of the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4 as the (D) ultraviolet absorber.

In the present invention, the dental curable composition may comprise a terephthalic acid ester compound represented by formula (3) as the (C) fluorescent agent.

[Formula (3)]

[Chemical formula 2]

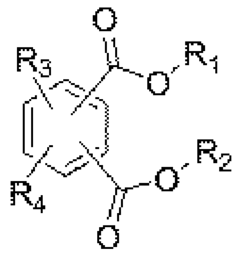

(In the formula, $R_1$ and $R_2$ each independently represent an alkyl group, $R_3$ represents a hydrogen atom, an amino group or a hydroxyl group, and $R_4$ represents an amino group or a hydroxyl group. The alkyl group has 1 to 3 carbon atoms, such as a methyl group, an ethyl group, an n-propyl group, or an i-propyl group.)

In the present invention, the dental curable composition may comprise only a terephthalic acid ester compound represented by formula (3) as the (C) fluorescent agent.

[Formula (3)]

[Chemical formula 3]

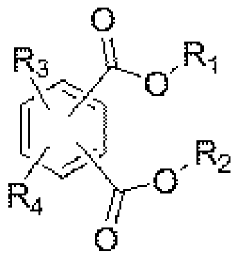

(In the formula, $R_1$ and $R_2$ each independently represent an alkyl group, $R_3$ represents a hydrogen atom, an amino group or a hydroxyl group, and $R_4$ represents an amino group or a hydroxyl group. The alkyl group has 1 to 3 carbon atoms, such as a methyl group, an ethyl group, an n-propyl group, or an i-propyl group.)

In the present invention, the dental curable composition may comprise, with respect to 100 parts by mass of the (A) polymerizable monomer, 0.5 to 10 parts by mass of the (B) polymerization initiator, 0.001 to 0.1 parts by mass of the(C) fluorescent agent, and 0.01 to 2 parts by mass of the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4.

Hereinafter, each component in the dental curable composition of the present invention is described in detail. The present invention is applied as a dental adhesive material, a dental composite resin, a dental core build-up material, a dental resin cement, a dental coating material, a dental sealant material, a dental manicure material, a dental splinting material, a dental glass ionomer cement, a dental hard resin, a dental cutting and machining material, a dental 3D printer material, a dental orthodontic material and the like.

In a dental practice, in order to restore aesthetically and functionally a lost portion of a tooth by caries, breakages and the like, a direct method which performs restoration with a dental adhesive material and composite resin and an indirect method which restores a prosthetic device consisting of ceramics or dental hard resin with a dental resin cement have been performed as treatment. In addition, a dental adhesive material for mounting a dental composite resin and various dental materials and a natural tooth, a dental splinting material for fixing a mobile tooth, a dental coating material for protecting a vital tooth after forming against a hyperesthesia, an external stimulation and secondary caries, a dental sealant material for preventing caries by filling deep fissures of a molar tooth, a dental manicure material for temporary recovering aesthetic property by masking discoloration of a tooth, and a dental core build-up material for forming an abutment tooth in the case of collapsing of a dental crown due to caries have been used. In recent years, new composite materials such as a dental cutting and machining material for preparing a prosthetic device by CAD/CAM processing, a dental 3D printer material for preparing a prosthetic device by 3D printer and a dental orthodontic material used for treating malocclusion have been developed, and various dental materials have been used for treatment. The above-described materials are prepared into a uniform paste by mixing a resin matrix consisting of several kinds of polymerizable monomers, a filler such as an inorganic filler and an organic-inorganic composite filler, and a polymerization initiator, according to the application. As one example of some materials, a dental composite resin for filling is used by filling into a tooth in the form of uncured paste, imparting anatomical form of a natural tooth with a dental instrument such as an instrument, and curing by irradiating light with a dental light irradiator or the like. For the irradiation light from a light irradiator, a light source having an output of about 100 to 3500 $mW/cm^2$ in the wavelength region of about 360 to 500 nm is generally used. On the other hand, a dental resin cement is used for adhering a prosthetic device to a tooth cavity or an abutment tooth, and is cured by light irradiation after attaching the prosthetic device to the tooth cavity or the abutment tooth.

As the photopolymerization initiator used in dental materials, a system of a photosensitizer and a system in which a photosensitizer are combined with an appropriate photopolymerization accelerator have been widely used. As the photosensitizer, acylphosphine oxide compounds and α-diketone compounds are known, and in particular, α-diketone compounds have an ability to initiate polymerization in the wavelength region of visible light which has little effect on the human body. Further, as a compound to be combined with a photosensitizer, a photoacid generator and a tertiary amine compound are well known. A combination of an α-diketone compound, a photoacid generator and a tertiary amine compound has high polymerization activity with respect to irradiation light, and thus has been used in a dental material field. The dental curable composition containing this photopolymerization initiator exhibits excellent mechanical properties such as hardness, flexural strength and compressive strength required for various materials.

As typical tertiary amine compounds used as such photoinitiators, aliphatic tertiary amines such as methyl diethanolamine, triethanolamine and dimethylaminoethyl methacrylate, and aromatic tertiary amines such as ethyl dimethylamino benzoate and p-tolylethanolamine are known. Only the combination of a photosensitizer, a photoacid generator and an aromatic tertiary amine compound does not sufficiently satisfy the various properties required for dental materials such as sufficient mechanical strength, sensitivity to light and color tone stability, but photopolymerization initiators consisting of the combination of a photosensitizer, a photoacid generator and an aliphatic tertiary amine compound may satisfy the various properties required for dental materials in a well-balanced manner and therefore are suitable for dental materials.

However, the dental curable compositions containing these photopolymerization initiators have a problem in that the cured body is significantly discolored when exposed to ultraviolet light contained in sunlight or the like. When the polymerized cured body discolors, the color tone compatibility with the surrounding tooth substance decreases tocause a problem that aesthetic property is imparied. Therefore, in JIS T 6514 and ISO 4049, tests for sunlight stability are spesified and the cured body is required not to show significant discoloration even when exposed to ultraviolet light for long periods of time.

In order to prevent discoloration of the cured body due to exposure to ultraviolet light, an ultraviolet absorber may be compounded into a dental curable composition. Known examples of ultraviolet absorbers that can be compounded in a dental curable composition include benzophenone-based ultraviolet absorbers, benzotriazole-based ultraviolet absorbers and triazine-based ultraviolet absorbers. Among these, benzotriazole-based ultraviolet absorbers are known to have excellent sunlight stability.

Furthermore, because a natural tooth absorbs ultraviolet light and emits fluorescence, a dental curable composition also may contain a fluorescent agent. Among fluorescent agents, phthalate ester-based fluorescent agents are commonly used. These fluorescent agents emit fluorescence by absorbing ultraviolet light in the wavelength region of 320 to 400 nm. Therefore, when these fluorescent agents are compounded with the above described ultraviolet absorbers, the ultraviolet light absorption of the fluorescent agent is inhibited to cause a problem of weakened fluorescence. In particular, most benzotriazole-based ultraviolet absorbers have the largest absorption maximum in the near ultraviolet region of 320 to 400 nm within the wavelength region of 250 to 400 nm and therefore have a problem of easily inhibiting fluorescence. When the amount of an ultraviolet absorber is reduced or the amount of an fluorescent agent is increased in order to compensate for the fluorescence, the function of the ultraviolet absorber becomes insufficient to cause a problem of reduced sunlight stability.

In order to solve this problem, the present inventors investigated with using an ultraviolet absorber that does not have an absorption maximum in the near ultraviolet region of 320 to 400 nm, but has an absorption maximum in the wavelength region of 250 to 320 nm. As aresult, it has been found that although the absorption of the fluorescent agent in the near ultraviolet region is not inhibited and excellent fluorescence is exhibited, the ultraviolet absorption in the wavelength region of 320 to 400 nm is insufficient to decrease sunlight stability.

Furthermore, in order to achieve both sunlight stability and fluorescence, by using a first ultraviolet absorber having an absorption maximum in the wavelength range of 250 to 320 nm and a second ultraviolet absorber having an absorption maximum in the wavelength range of 320 to 400 nm, a combination of two or more types of ultraviolet absorbers having different absorption maximum wavelengths are compounded. However, as a result of investigations by the present inventors, it has been found that ultraviolet absorbers usually have low solubility with respect to polymerizable monomer, and when two or more types of ultraviolet absorbers are dissolved in a polymerizable monomer, the individual solubilities of those ultraviolet absorbers with respect to a polymerizable monomer differ, and therefore a problem is recognized in further reduction in solubility with respect to a polymerizable monomer compared to the case where each ultraviolet absorber is compounded alone. As a result, the time required for production becomes longer, and a phenomenon such as uneven coloration of a curable composition has been observed in the case where the dissolution of ultraviolet absorber with respect to a polymerizable monomer is insufficient.

As a result of further investigations, the present inventors have found that by using the compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4 as an ultraviolet absorber, it is possible to obtaine a dental curable composition having excellent sunlight stability, fluorescence and mechanical property. It has been also found to exhibit effects such as eliminating the need to compound multiple ultraviolet absorbers, preventing uneven coloring by good solubility with respect to a polymerizable monomer and short production time. The present invention is based on these findings.

The dental curable composition of the present invention has excellent sunlight stability, fluorescence, and mechanical strength. Furthermore, since the ultraviolet absorber in the present invention has excellent solubility with respec to a polymerizable monomer, it is possible to achieve the effects such as shortening production time and reducing the occurrence of uneven coloring.

Next, a specific embodiment of the present invention will be described.

[(A) Polymerizable monomer]

As the (A) polymerizable monomer contained in the dental curable composition of the present invention, any polymerizable monomers can be used without limitation as long as it is known. In the polymerizable monomer or the compound having a polymerizable group described in the present invention, the polymerizable group preferably exhibits radical polymerizability, and specifically, from the viewpoint of easy radical polymerization, the polymerizable group is preferably (meth) acrylic group and/or (meth) acrylamide group. In the present specification, "(meth) acrylic" means acrylic and/or methacrylic, "(meth) acryloyl" means acryloyl and/ or methacryloyl, "(meth) acrylate" means acrylate and/or methacrylate, and, "(meth) acrylamide" means acrylamide and/or methacrylamide. A polymerizable monomer having a substituent at the $\alpha$-position of an acrylic group and/or an acrylamide group can also be preferably used. Specific examples include one having one radical polymerizable group, one having two radical polymerizable groups, one having three or more radical polymerizable groups, one having an acidic group, one having an alkoxysilyl group, and one having a sulfur atom.

Specific examples of a polymerizable monomer having one radical polymerizable group and not containing acidic group include 2-hydroxyethyl (meth) acrylate, 3-hydroxypropyl (meth) acrylate, 4-hydroxybutyl (meth) acrylate, 2-hydroxypropyl (meth) acrylate, 2-hydroxybutyl (meth) acrylate, 6-hydroxyhexyl (meth) acrylate, 10-hydroxydecyl (meth) acrylate, propylene glycol mono (meth) acrylate, glycerol mono (meth) acrylate, erythritol mono (meth) acrylate, N-methylol (meth) acrylamide, N-hydroxyethyl (meth) acrylamide, N,N-(dihydroxyethyl) (meth) acrylamide, methyl (meth) acrylate, ethyl (meth) acrylate, propyl (meth) acrylate, isopropyl (meth) acrylate, butyl (meth) acrylate, isobutyl (meth) acrylate, benzyl (meth) acrylate, lauryl (meth) acrylate, 2,3-dibromopropyl (meth) acrylate, 3-(meth) acryloyloxypropyl trimethoxysilane, 11-(meth) acryloyloxyundecyl trimethoxysilane, (meth) acrylamide and the like.

Specific Examples of the polymerizable monomer having two radical polymerizable groups and not containing acidic group include 2,2-bis ((meth) acryloyloxy phenyl) propane, 2,2-bis [4-(3-(meth) acryloyloxy)-2-hydroxy propoxyphenyl] propane (generally called "Bis-GMA"), 2,2-bis (4-(meth) acryloyloxy phenyl) propane, 2,2-bis (4-(meth) acryloyloxy polyethoxyphenyl) propane, 2,2-bis (4-(meth) acryloyloxy diethoxyphenyl) propane, 2,2-bis (4-(meth) acryloyloxy tetraethoxyphenyl) propane, 2,2-bis (4-(meth)) acryloyloxy pentaethoxyphenyl) propane, 2,2-bis (4-(meth) acryloyloxy dipropoxyphenyl) propane, 2-(4-(meth) acryloyloxy diethoxyphenyl)-2-(4-(meth) acryloyloxy diethoxyphenyl) propane, 2-(4-(meth) acryloyloxy diethoxyphenyl)-2-(4-(meth) acryloyloxy ditriethoxyphenyl) propane, 2-(4-(meth) acryloyloxy dipropoxyphenyl)-2-(4-(meth) acryloyloxy triethoxyphenyl) propane, 2,2-bis (4-(meth) acryloyloxy propoxyphenyl) propane, 2,2-bis (4-(meth) acryloyloxy isopropoxyphenyl) propane, 1,4-bis (2-(meth) acryloyloxyethyl) pyromellitate, glycerol di (meth) acrylate, 1-(acryloyloxy)-3-(methacryloyloxy)-2-propanol, ethyleneglycol di (meth) acrylate, diethyleneglycol di (meth) acrylate, triethylene glycol di (meth) acrylate, propylene glycol di (meth) acrylate, butylene glycol di (meth) acrylate, neopentyl glycol di (meth) acrylate, polyethylene glycol di (meth) acrylate, 1,3-butanediol di (meth) acrylate, 1,5-pentanediol di (meth) acrylate, 1,6-hexanediol di (meth) acrylate, 1,10-decanediol di (meth) acrylate, 1,2-bis (3-methacryloyloxy-2-hydroxypropoxy) ethane, 2,2,4-trimethyl hexamethylene bis (2-carbamoyloxy ethyl) dimethacrylate (generally called "UDMA"), 1,2-bis (3-methacryloyloxy-2-hydroxy propoxy) ethane and the like.

Specific Examples of the polymerizable monomer having three or more radical polymerizable groups and not containing acidic group include trimethylolpropane tri (meth) acrylate, trimethylolethane tri (meth) acrylate, trimethylolmethane tri (meth) acrylate, pentaerythritol tri (meth) acrylate, pentaerythritol tetra (meth) acrylate, dipentaerythritol penta (meth) acrylate, N,N-(2,2,4-trimethyl hexamethylene) bis [2- (aminocarboxy) propane-1,3-diol] tetra methacrylate, 1,7-diacryloyloxy-2,2,6,6-tetra acryloyloxymethyl-4-oxyheptane and the like.

Specific examples of the polymerizable monomer having an alkoxysilyl gruop include a (meth) acrylic compound and a (meth) acrylamide compound having one alkoxysilyl group in the molecule and a (meth) acrylic compound and a (meth) acrylamide compound having a plurality of alkoxysilyl groups in the molecule. Specific examples include 2-(meth) acryloxyethyl trimethoxysilane, 3-(meth) acryloxypropyl trimethoxysilane, 3-(meth) acryloxypropyl triethoxysilane, 3-(meth) acryloxypropyl methyldimethoxysilane, 4-(meth) acryloxybutyl trimethoxysilane, 5-(meth) acryloxypentyl trimethoxysilane, 6-(meth) acryloxyhexyl trimethoxysilane, 7-(meth) acryloxyheptyl trimethoxysilane, 8-(meth) acryloxyoctyl trimethoxysilane, 9-(meth) acryloxynonyl trimethoxysilane, 10-(meth) acryloxydecyl trimethoxysilane, 11-(meth) acryloxyundecyl trimethoxysilane. Furthermore, specific examples having an urethane group or an ether group include 3,3-dimethoxy-8,37-dioxo-2,9,36-trioxa-7,38-diaza-3-silatetracontan-40-yl (meth) acrylate, 2-((3,3-dimethoxy-8-oxo-2,9,18-trioxa-7-aza-3-silanonadecane-19-oyl) amino)-2-methylpropane-1,3-diyl di (meth) acrylate, 3,3-dimethoxy-8,19-dioxo-2,9,18-trioxa-7,20-diaza-3-siladocosane-22-yl (meth) acrylate, 3,3-dimethoxy-8,22-dioxo-2,9,12,15,18,21-hexaoxa-7,23-diaza-3-silapentacosane-25-yl (meth) acrylate, 3,3-dimethoxy-8,22-dioxo-2,9,12,15,18,21,26-heptaoxa-7,23-diaza-3-silaoctacosane-28-yl (meth) acrylate, 3,3-dimethoxy-8,19-dioxo-2,9,12,15,18-pentaoxa-7,20-diaza-3-siladocosane-22-yl (meth) acrylate, 3,3-dimethoxy-8,19-dioxo-2,9,12,15,18,23-hexaoxa-7,20-diaza-3-silapentacosane-25-yl (meth) acrylate, 2-((3,3-dimethoxy-8-oxo-2,9,12,15,18-pentaoxa-7-aza-3-silanonadecane-19-oyl) amino) -2-methylpropan-1,3-diyldi (meth) acrylate, 4,4-diethoxy-17-oxo-3,16,21-trioxa-18-aza-4-silatricosane-23-yl (meth) acrylate, 4,4-diethoxy-17-oxo-3,16,21,24-tetraoxa-18-aza-4-silahexacosane-26-yl (meth) acrylate, 4,4-diethoxy-13-oxo-3,12,17-trioxa-14-aza-4-silanonadecane-19-yl (meth) acrylate, 4,4-diethoxy-17-oxo-3,16-dioxa-18-aza-4-silaicosane-20-yl (meth) acrylate and 2-methyl-2-((11-(triethoxysilyl) undecyloxy) carbonylamino) propan-1,3-diyldi (meth) acrylate.

The dental curable composition of the present invention may contain a polymerizable monomer having a sulfur atom as the (A) polymerizable monomer in order to impart adhesive property with respect to a noble metal. As the polymerizable monomer having a sulfur atom, any known compound can be used without any limitation as long as it is a polymerizable monomer having one or more sulfur atoms and a polymerizable group. Specifically, it refers to a compound having a partial structure such as -SH, -S-S-, >C=S, >C-S-C<, >P=S, or a compound prepared by tautomerism. Specific examples include 10-methacryloxy decyl-6,8-dithiooctanate, 6-methacryloxy hexyl-6,8-dithiooctanate, 6-methacryloxy hexyl-2-thiouracil-5-carboxylate, 2-(11-methacryloxy undecylthio)-5-mercapto-1,3,4-thiadiazole, 10-(meth) acryloxy decyl dihydrogenthiophosphate.

An oligomer or a prepolymer having at least one polymerizable group in its molecule may be used other than such a polymerizable monomer, without any limitation. There is no problem even if a substituent such as a fluoro group is contained in the same molecule. The polymerizable monomers described above can be used not only singly but also in combinations of a plurality thereof.

The dental curable composition of the present invention may contain a silane coupling agent as the (A) polymerizable monomer in order to impart adhesive property with respect to glass ceramics. Any known silane coupling agent can be used without any limitation, but 3-methacryloxypropyl trimethoxysilane, 8-methacryloxyoctyl trimethoxysilane, and 11-methacryloxyundecyl trimethoxysilane and the like are preferable. From the viewpoint of imparting adhesive property, the compounding amount is, with respect to 100 parts by mass of the total amount of the polymerizable monomer contained in the composition, preferably 1 part by mass or more, more preferably 5 parts by mass or more and less than 20 parts by mass. Since the purpose of the silane coupling agent as a polymerizable monomer is to impart adhesive property with respect to glass ceramics or a resin material containing a filler consisting of glass ceramics, the silane coupling agent is compounded separately from the surface treatment agent of the filler.

The dental curable composition of the present invention may contain a polymerizable monomer having a sulfur atom as the (A) polymerizable monomer in order to impart adhesive property with respect to a noble metal. From the view point of imparting adhesive property, the compounding amount of the polymerizable monomer having a sulfur atom is, with respect to 100 parts by mass of the total amount of the polymerizable monomer contained in the dental curable composition, 0.01 parts by mass or more, preferably 0.1 parts by mass or more and less than 20 parts by mass.

There is no problem even if a polymerizable monomer having a cationic polymerizable functional group is contained as the polymerizable monomer contained in the dental curable composition of the present invention, but it is preferable to contain only a polymerizable monomer having a radical polymerizable functional group. When a cationic polymerizable monomer is contained, there is a case where storage stability decrease.

Specific examples of the polymerizable monomer having a cationic polymerizable functional group include a vinyl ether compound, an epoxy compound, an oxetane compound, a cyclic ether compound and a cyclic carbonate compound.

The dental curable composition of the present invention may contain (A1) polymerizable monomer having an acidic group. For the polymerizable monomer having an acidic group, any polymerizable monomer can be used without any limitation as long as it has one or more polymerizable group and at least one acidic group such as a phosphoric acid group, a pyrophosphoric acid group, a thiophosphoric acid group, a phosphonic acid group, a sulfonic acid group and a carboxylic acid group and the like. It is possible to impart adhesive property with respect to a tooth substance and a prosthetic device by containing a polymerizable monomer having an acidic group.

Specific examples of a phosphoric acid group-containing polymerizable monomer include 2-(meth) acryloyloxyethyl dihydrogen phosphate, 3-(meth) acryloyloxypropyl dihydrogen phosphate, 4-(meth) acryloyloxybutyl dihydrogen phosphate, 5-(meth) acryloyloxypentyl dihydrogen phosphate, 6-(meth) acryloyloxyhexyl dihydrogen phosphate, 7-(meth) acryloyloxyheptyl dihydrogen phosphate, 8-(meth) acryloyloxyoctyl dihydrogen phosphate, 9-(meth) acryloyloxynonyl dihydrogen phosphate, 10-(meth) acryloyloxydecyl dihydrogen phosphate, 11-(meth) acryloyloxyundecyl dihydrogen phosphate, 12-(meth) acryloyloxydodecyl dihydrogen phosphate, 16-(meth) acryloyloxyhexadecyl dihydrogen phosphate, 20-(meth) acryloyloxyicosyl dihydrogen phosphate, bis [2-(meth) acryloyl oxyethyl] hydrogensphosphate, bis [4-(meth) acryloyl oxybutyl] hydrogen phosphate, bis [6-(meth) acryloyl oxyhexyl] hydrogen phosphate, bis [8-(meth) acryloyl oxyoctyl] hydrogen phosphate, bis [9-(meth) acryloyl oxynonyl] hydrogen phosphate, bis [10-(meth) acryloyl oxydecyl] hydrogen phosphate, 1,3-di (meth) acryloyl oxypropyl dihydrogenphosphate, 2-(meth) acryloyl oxyethylphenyl hydrogen phosphate, 2-(meth) acryloyloxyethyl-2-bromoethyl hydrogen phosphate and bis [2-(meth) acryloyloxy-(1-hyrdoxymethyl) ethyl] hydrogen phosphate; acyl chloride, alkali metal salt and ammonium salt thereof; and (meth) acrylamide compound in which the ester bond of these compounds is substituted with an amide bond, and the like.

Specific examples of a pyrophosphoric acid group-containing polymerizable monomer include bis [2-(meth) acryloyl oxyethyl] pyrophosphate, bis [4-(meth) acryloyl oxybutyl] pyrophosphate, bis [6-(meth) acryloyl oxyhexyl] pyrophosphate, bis [8-(meth) acryloyl oxyoctyl] pyrophosphate, bis [10-(meth) acryloyl oxydecyl] pyrophosphate; acyl chloride, alkali metal salt and ammonium salt thereof; and (meth) acrylamide compound in which the ester bond of these compounds

is substituted with an amide bond, and the like.

Specific examples of a thiophosphate group-containing polymerizable monomer include 2-(meth) acryloyloxyethyl dihydrogen thiophosphate, 3-(meth) acryloyloxypropyl dihydrogen thiophosphate, 4-(meth) acryloyloxybutyl dihydrogen thiophosphate, 5-(meth) acryloyloxypentyl dihydrogen thiophosphate, 6-(meth) acryloyloxyhexyl dihydrogen thiophosphate, 7-(meth) acryloyloxyheptyl dihydrogen thiophosphate, 8-(meth) acryloyloxyoctyl dihydrogen thiophosphate, 9-(meth) acryloyloxynonyl dihydrogen thiophosphate, 10-(meth) acryloyloxydecyl dihydrogen thiophosphate, 11-(meth) acryloyloxyundecyl dihydrogen thiophosphate, 12-(meth) acryloyloxydodecyl dihydrogen thiophosphate, 16-(meth) acryloyloxyhexadecyl dihydrogen thiophosphate, 20-(meth) acryloyloxyicosyl dihydrogen thiophosphate; acyl chloride, alkali metal salt and ammonium salt thereof; and (meth) acrylamide compound in which the ester bond of these compounds is substituted with an amide bond, and the like. The polymerizable monomer having a thiophosphate group is also classified as a polymerizable monomer having a sulfur atom.

Specific examples of a phosphonic acid group-containing polymerizable monomer include 2-(meth) acryloyloxy ethylphenyl phosphonate, 5-(meth) acryloyloxy pentyl-3-phosphonopropionate, 6-(meth) acryloyloxy hexyl-3-phosphonopropionate, 10-(meth) acryloyloxy decyl-3-phosphonopropionate, 6-(meth) acryloyloxy hexyl-3-phosphonoacetate, 10-(meth) acryloyloxy decyl-3-phosphonoacetate; acyl chloride, alkali metal salt and ammonium salt thereof; and (meth)acrylamide compound in which the ester bond of these compounds is substituted with an amide bond, and the like.

Specific examples of a sulfonic acid group-containing polymerizable monomer include 2-(meth) acrylamide-2-methyl propanesulfonic acid and 2-sulfoethyl (meth) acrylate and the like.

The carboxylic acid group-containing polymerizable monomers are classified into a (meth) acrylic-based compound having one carboxyl group in the molecule and a (meth) acrylic-based compound having a plurality of carboxyl groups in the molecule. Examples of the (meth) acrylic-based compound having one carboxyl group in the molecule include (meth) acrylic acid, N-(meth) acryloyl glycine, N-(meth) acryloyl aspartic acid, O-(meth) acryloyl tyrosine, N-(meth) acryloyl tyrosine, N-(meth) acryloyl phenylalanine, N-(meth) acryloyl-p-aminobenzoic acid, N-(meth) acryloyl-o-aminobenzoic acid, p-vinylbenzoic acid, 2-(meth) acryloyloxybenzoic acid, 3-(meth) acryloyloxybenzoic acid, 4-(meth) acryloyloxybenzoic acid, N-(meth) acryloyl-5-aminosalicylic acid, N-(meth) acryloyl-4-aminosalicylic acid, 2-(meth) acryloyloxyethyl hydrogen succinate, 2-(meth) acryloyloxyethyl hydrogen phthalate, 2-(meth) acryloyloxyethyl hydrogenmalate; acyl chloride thereof; and (meth)acrylamide compound in which the ester bond of these compounds is substituted with an amide bond, and the like. Examples of the (meth) acrylic-based compound having a plurality of carboxyl groups in the molecule include 6-(meth) acryloyl oxyhexane-1,1-dicarboxylic acid, 9-(meth) acryloyl oxynonane-1,1-dicarboxylic acid, 10-(meth) acryloyl oxydecane-1,1-dicarboxylic acid, 11-(meth) acryloyloxy undecane-1,1-dicarboxylic acid, 12-(meth) acryloyl oxydodecane-1,1-dicarboxylic acid, 13-(meth) acryloyloxy tridecane-1,1-dicarboxylic acid, 4-(meth) acryloyloxyethyl trimeritate, 4-(meth) acryloyloxybutyl trimeritate, 4-(meth) acryloyloxyhexyl trimeritate, 4-(meth) acryloyloxydecyl trimeritate, 2-(meth) acryloyl oxyethyl-3'-(meth) acryloyloxy-2'-(3,4-dicarboxy benzoyloxy) propylsuccinate; acid anhydrides and acid halides thereof,; and (meth) acrylamide compound in which the ester bond of these compounds is substituted with an amide bond, and the like.

Preferable examples of the (A1) polymerizable monomer having an acidic group include (meth) acrylic acid, 10-methacryloyloxydecyl dihydrogenphosphate, 6-methacryloxyhexyl phosphonoacetate, 4-(meth) acryloyloxyethyl trimeritate and their acid anhydrides. From the view point of imparting adhesive property, the compounding amount of the polymerizable monomer having an acidic group is preferably 1 part by mass or more and 20 parts by mass or less with respect to 100 parts by mass the (A) polymerizable monomer. When the compounding amount is less than 1 part by mass, there is a case where sufficient adhesive property is not exhibited. When the compounding amount is more than 20 parts by mass, there is a case where a decrease in storage stability or a decrease in curability occurs.

[(B) Polymerization initiator]

As the polymerization initiator used in the dental curable composition of the present invention, it is not particularly limited, and any known radical generator can be used. Polymerization initiators are generally classified into the polymerization initiator that initiates polymerization by mixing the same with the monomers immediately before use (chemical polymerization initiator), the polymerization initiator that initiates polymerization by heating or warming (thermal polymerization initiator), and the polymerization initiator that initiates polymerization by light irradiation (photopolymerization initiator), and can be selected from these according to the method and purpose of use. In addition, these polymerization initiators can be used not only singly but also in combinations of two or more, regardless of the polymerization manner or the polymerization method. Furthermore, these polymerization initiators can be used without any problem even if they are subjected to secondary treatment such as encapsulation in microcapsules in order to stabilize or delay the polymerization.

Examples of the chemical polymerization initiator include a redox-type polymerization initiator system comprising an organic peroxide/an amine compound, an organic peroxide/an amine compound/a sulfinate, an organic peroxide/an amine compound/a borate compound, and an organometallic type polymerization initiator system which initiate polymerization by reacting with oxygen or water. Further, sulfinates and borate compounds can initiate polymerization by reacting with a polymerizable monomer having an acidic group.

Specific examples of the organic peroxide include benzoyl peroxide, parachlorobenzoyl peroxide, 2,4-dichlorobenzoyl peroxide, acetyl peroxide, lauroyl peroxide, tertiary-butyl peroxide, cumenehydro peroxide, 2,5-dihydro peroxide, methylethylketone peroxide, and tertiary-butylperoxybenzoade.

As specific examples of the amine compound, secondary and tertiary amine in which an amino group bonds to an aryl group are preferable, and specific examples thereof include p-N,N-dimethyl-toluidine, N,N-dimethylaniline, N-β-hydroxyethyl-aniline, N,N-di (P-hydroxyethyl)-aniline, p-N,N-di (P-hydroxyethyl)-toluidine, N-methyl-aniline, and p-N-methyl-toluidine.

Specific examples of the sulfinates include sodium benzenesulfinate, lithium benzenesulfinate, and sodium p-toluenesulfinate.

Specific examples of the borate compound include sodium salts, lithium salts, potassium salts, magnesium salts, tetrabutylammonium salts, and tetramethylammonium salts of trialkylphenyl borone and trialkyl (p-phlorophenyl) borone (alkyl groups are selected from an n-butyl group, an n-octyl group, an n-dodecyl group, etc.).

Specific examples of the organometal type polymerizable initiator include organic boron compounds such as triphenylborane, tributylborane and a partially oxidized tributylborane, and the transition metal compounds of the group 4 in the periodic table.

Examples of the thermal polymerization initiator capable of initiating polymerization by heating or warming include azo compounds such as azobisisobutyronitrile and methyl azobisisobutyrate azobiscyanovaleric acid are preferably used, in addition to the above organic peroxide.

Examples of the photopolymerization initiator include those consisting of a photosensitizer, a combination of a photosensitizer/photopolymerization accelerator, and a combination of a photosensitizer/photoacid generator/photopolymerization accelerator. Among these, the photopolymerization initiator used in the dental curable composition of the present invention preferably contains a combination of a (B-1) photosensitizer, a (B-2) photoacid generator, and a (B-3) polymerization accelerator. These are not particularly limited, and any known compounds commonly used may be used without any limitation.

[(B-1) Photosensitizer]

Specific examples of the (B-1) photosensitizer which can be used in the present invention include α-diketones such as benzil, camphorquinone, camphorquinone carboxylic acid, camphorquinone sulfonic acid, α-naphthyl, acetonaphthene, p,p'-dimethoxybenzyl, p,p'-dichlorobenzylacetyl, pentanedion, 1,2-phenanthrenequinone, 1,4-phenanthrenequinone, 3,4-phenanthrenequinone, 9,10-phenanthrenequinone and naphthoquinone; benzoin alkyl ethers such as benzoin, benzoin methyl ether and benzoin ethyl ether; thioxanthones such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone; benzophenones such as benzophenone, p-chlorobenzophenone and p-methoxybenzophenone; acylphosphine oxides such as bis (2,6-dimethoxy benzoyl) phenylphosphine oxide, bis (2,6-dimethoxy benzoyl) (2,4,4-trimethyl pentyl) phosphine oxide, bis (2,6-dimethoxy benzoyl)-n-butylphosphine oxide, bis (2,6-dimethoxy benzoyl)-(2-methylprop-1-yl) phosphine oxide, bis (2,6-dimethoxy benzoyl)-(1-methylprop-1-yl) phosphine oxide, bis (2,6-dimethoxy benzoyl)-t-butyl phosphine oxide, bis (2,6-dimethoxy benzoyl) cyclohexyl phosphine oxide, bis (2,6-dimethoxy benzoyl) octyl phosphine oxide, bis (2-methoxy benzoyl) (2-methylprop-1-yl) phosphine oxide, bis (2-methoxy benzoyl) (1-methylprop-1-yl) phosphine oxide, bis (2,6-diethoxy benzoyl) (2-methylprop-1-yl) phosphine oxide, bis (2,6-diethoxy benzoyl) (1-methylprop-1-yl) phosphine oxide, bis (2,6-dibutoxy benzoyl) (2-methylprop-1-yl) phosphine oxide, bis (2,4-dimethoxy benzoyl) (2-methylprop-1-yl) phosphine oxide, bis (2,4,6-trimethyl benzoyl) phenyl phosphine oxide, 2,4,6-trimethyl benzoyl diphenyl phosphine oxide, bis (2,4,6-trimethyl benzoyl) (2,4-dipentoxy phenyl) phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, 2,6-dimethoxy benzoyl benzyl butyl phosphine oxide, 2,6-dimethoxy benzoyl benzyl octyl phosphine oxide, bis (2,4,6-trimethyl benzoyl) isobutyl phosphine oxide and 2,6-dimethoxy benzoyl-2,4,6-trimethyl benzoyl-n-butyl phosphine oxide; acylgermanium compounds such as bisbenzoyl diethylgermanium, bisbenzoyl dimethylgermanium, bisbenzoyl dibutylgermanium, bis (4-methoxybenzoyl) dimethylgermanium and bis (4-methoxybenzoyl) diethylgermanium; α-aminoacetophenones such as 2-benzyl-dimethylamino-1-(4-morpholinophenyl)-butanone-1, and 2-benzyl-diethylamino-1-(4-morpholinophenyl)-propanone-1; ketals such as benzyl dimethyl ketal, benzyl diethyl ketal and benzyl (2-methoxyethyl ketal); and titanocenes such as bis (cyclopentadienyl)-bis [2,6-difluoro-3-(1-pyrrolyl) phenyl]-titanium, bis (cyclopentadienyl)-bis (pentanefluorophenyl)-titanium and bis (cyclopentadienyl)-bis (2,3,5,6-tetrafluoro-4-disiloxyphenyl)-titanium.

The photosensitizer (B-1) may be appropriately selected according to the wavelength, the intensity and the irradiation time of light used for polymerization, and the type and the compounding amount of other components to be

combined. In addition, the photosensitizer may be used not only singly but also in combinations of two or more. Among them, α-diketone compounds having a maximum absorption wavelength in the visible light region are preferably used, and camphorquinone compounds such as camphorquinone, camphorquinone carboxylic acid and camphorquinone sulfonic acid are more preferable. Camphorquinone is particularly preferred because it is easily available.

The dental curable composition of the present invention may contain only α-diketone compounds as the (B-1) photosensitizer.

[(B-2) Photoacid generator]

As the (B-2) photoacid generator used in the dental curable composition of the present invention, known compounds can be used without limitation. Specific examples include triazine compounds, iodonium salt compounds, sulfonium salt compounds, and sulfonic acid ester compounds. Among these, triazine compounds and iodonium salt-based compounds are preferable because of having high polymerizability in the case of using in combination with a sensitizer. Iodonium salt-based compounds are more preferable. Iodonium-based salt compounds are susceptible to sensitization by photosensitizers that have absorption in the visible light region.

Specific examples of the triazine compound include 2,4,6-tris (trichloro methyl)-s-triazine, 2,4,6-tris (tribromo methyl)-s-triazine, 2-methyl-4,6-bis (trichloro methyl)-s-triazine, 2-methyl-4,6-bis (tribromo methyl)-s-triazine, 2-phenyl-4,6-bis (trichloro methyl)-s-triazine, 2-(p-methoxy phenyl)-4,6-bis (trichloro methyl)-s-triazine, 2-(p-methyl thiophenyl)-4,6-bis (trichloro methyl)-s-triazine, 2-(p-chloro phenyl)-4,6-bis (trichloro methyl)-s-triazine, 2-(2,4-dichloro phenyl)-4,6-bis (trichloro methyl)-s-triazine, 2-(p-bromo phenyl)-4,6-bis (trichloro methyl)-s-triazine, 2-(p-tolyl)-4,6-bis (trichloro methyl)-s-triazine, 2-n-propyl-4,6-bis (trichloro methyl)-s-triazine, 2-(α,α,β-trichloro ethyl)-4,6-bis (trichloro methyl)-s-triazine, 2-styryl-4,6-bis (trichloro methyl)-s-triazine, 2-[2-(p-methoxy phenyl) ethenyl]-4,6-bis (trichloro methyl)-s-triazine, 2-[2-(o-methoxy phenyl) ethenyl]-4,6-bis (trichloro methyl)-s-triazine, 2-[2-(p-butoxy phenyl) ethenyl]-4,6-bis (trichloro methyl)-s-triazine, 2-[2-(3,4-dimethoxy phenyl) ethenyl]-4,6-bis (trichloro methyl)-s-triazine, 2-[2-(3,4,5-trimethoxy phenyl) ethenyl]-4,6-bis (trichloro methyl)-s-triazine, 2-(1-naphthyl)-4,6-bis (trichloro methyl)-s-triazine, 2-(4-biphenylyl)-4,6-bis (trichloro methyl)-s-triazine, 2-[2-{N,N-bis(2-hydroxy ethyl) amino} ethoxy]-4,6-bis (trichloro methyl)-s-triazine, 2-[2-{N-hydroxy ethyl-N-ethylamino} ethoxy]-4,6-bis (trichloro methyl)-s-triazine, 2-[2-{N-hydroxy ethyl-N-methylamino} ethoxy]-4,6-bis (trichloro methyl)-s-triazine, 2-[2-{N,N-diallyl amino} ethoxy]-4,6-bis (trichloro methyl)-s-triazine and the like. Among them, 2,4,6-tris (trichloro methyl)-s-triazine is preferable.

Any iodonium salt-based compound can be used as long as it is known. For the specific examples, the structural formula of the iodonium salt-based compound can be represented by the following formula (2).

[Formula (2)] $[(R1)_2I]^+ [A]^-$

(In the formula, $[(R1)_2I]^+$ is a cation part, $[A]^-$ is an anion part, R1 shown in the formula (2) represents an organic group bonded to I, and R1s may be the same or different. R1 represents, for example, an aryl group having 6 to 30 carbon atoms, a heterocyclic group having 4 to 30 carbon atoms, an alkyl group having 1 to 30 carbon atoms, an alkenyl group having 2 to 30 carbon atoms, or an alkynyl group having 2 to 30 carbon atoms, which may have at least one substituted group selected from the group consisting of groups such as alkyl, hydroxy, alkoxy, alkylcarbonyl, arylcarbonyl, alkoxycarbonyl, aryloxycarbonyl, arylthiocarbonyl, acyloxy, arylthio, alkylthio, aryl, heterocycle, aryloxy, alkylsulfinyl, arylsulfinyl, alkylsulfonyl, arylsulfonyl, alkyleneoxy, amino, cyano, nitro groups and halogens.)

In the above, examples of the aryl group having 6 to 30 carbon atoms include a monocyclic aryl group such as a phenyl group and a condensed polycyclic aryl group such as a naphthyl, anthrasenyl, phenanthrenyl, pyrenyl, chrysenyl, naphthacenyl, benzanthrasenyl, anthraquinolyl, fluorenyl, naphthoquinone and anthraquinone.

Examples of the heterocyclic group having 4 to 30 carbon atoms include cyclic groups containing 1 to 3 heteroatoms such as oxygen, nitrogen, and sulfur, which may be the same or different. Specific examples include a monocyclic heterocyclic group such as thienyl, furanyl, pyranyl, pyrrolyl, oxazolyl, thiazolyl, pyridyl, pyrimidyl and pyrazinyl, and a condensed polycyclic heterocyclic group such as indolyl, benzofuranyl, isobenzofuranyl, benzothienyl, isobenzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, dibenzothienyl, xanthonyl, thioxanthonyl and dibenzofuran.

Specific examples of the alkyl groups having 1 to 30 carbon atoms include a linear alkyl group such as methyl, ethyl, propyl, butyl, hexadecyl and octadecyl, a branched alkyl group such as isopropyl, isobutyl, sec-butyl, tert-butyl, isopentyl, neopentyl, tert-pentyl, isohexyl and a cycloalkyl groups such as cyclopropyl, cyclobutyl, cyclopentyl and cyclohexyl.

In addition, specific examples of the alkenyl group having 2 to 30 carbon atoms include a linear chain or branched group such as vinyl, allyl, 1-propenyl, isopropenyl, 1-butenyl, 2-butenyl, 3-butenyl and 1-methyl-1-propenyl.

Further, specific examples of the alkynyl group having 2 to 30 carbon atoms include a linear chain or branched group such as ethynyl, 1-propynyl, 2-propynyl, 1-butynyl, 2-butynyl, 3-butynyl, 1-methyl-1-propynyl and 1-methyl-2-propynyl.

The above-described aryl group having 6 to 30 carbon atoms, heterocyclic group having 4 to 30 carbon atoms, alkyl group having 1 to 30 carbon atoms, alkenyl group having 2 to 30 carbon atoms and alkynyl group having 2 to 30 carbon atoms may have at least one substituted group. Specific examples of the substituted group include a linear alkyl group having 1 to 18 carbon atoms such as methyl, ethyl, propyl, butyl and octadecyl; a branched alkyl group having 1 to 18 carbon atoms such as isopropyl, isobutyl, sec-butyl and tert- butyl; a cycloalkyl group having 3 to 18 carbon atoms such as cyclopropyl, cyclobutyl, cyclopentyl and cyclohexyl; a hydroxy group; a linear chain or branched alkoxy group having 1 to 18 carbon atoms such as methoxy, ethoxy, propoxy, isopropoxy, butoxy, isobutoxy, sec-butoxy, tert-butoxy and dodecyloxy; a linear chain or branched alkylcarbonyl group having 2 to 18 carbon atoms such as acetyl, propionyl, butanoyl, 2-methylpropionyl, heptanoyl, 2-methylbutanoyl, 3-methylbutanoyl and octanoyl; an arylcarbonyl group having 7 to 11 carbon atoms such as benzoyl and naphthoyl; a linear chain or branched alkoxycarbonyl group having 2 to 19 carbon atoms such as methoxycarbonyl, ethoxycarbonyl, propoxycarbonyl, isopropoxycarbonyl, butoxycarbonyl, isobutoxycarbonyl, sec-butoxycarbonyl and tert-butoxycarbonyl; an aryloxycarbonyl group having 7 to 11 carbon atoms such as phenoxycarbonyl and naphthoxycarbonyl; an arylthiocarbonyl group having 7 to 11 carbon atoms such as phenylthiocarbonyl and naphthoxythiocarbonyl; a linear chain or branched acyloxy group having 2 to 19 carbon atoms such as acetoxy, ethylcarbonyloxy, propylcarbonyloxy, isobutylcarbonyloxy, sec-butylcarbonyloxy, tert-butylcarbonyloxy and octadecylcarbonyloxy; an arylthio group having 6 to 20 carbon atoms such as phenylthio, biphenylthio, methylphenylthio, chlorophenylthio, bromophenylthio, fluorophenylthio, hydroxyphenylthio, methoxyphenylthio, naphthylthio, 4-[4-(phenylthio) benzoyl] phenylthio, 4-[4-(phenylthio) phenoxy] phenylthio, 4-[4-(phenylthio) phenyl] phenylthio, 4-(phenylthio) phenylthio, 4-benzoyl phenylthio, 4-benzoyl-chlorophenylthio, 4-benzoyl-methylthio phenylthio, 4-(methylthiobenzoyl) phenylthio and 4-(p-tert-butylbenzoyl) phenylthio; a linear chain or branched alkylthio group having 1 to 18 carbon atoms such as methylthio, ethylthio, propylthio, tert-butylthio, neopentylthio and dodecylthio; an aryl group having 6 to 10 carbon atoms such as phenyl, tolyl, dimethylphenyl and naphthyl; a heterocycle group having 4 to 20 carbon atoms such as thienyl, furanyl, pyranyl, xanthenyl, chromanyl, isochromanyl, xanthonyl, thioxanthonyl and dibenzofuranyl; an aryloxy group having 6 to 10 carbon atoms such as phenoxy and naphthyloxy; a linear chain or branched alkylsulfinyl group having 1 to 18 carbon atoms such as methylsulfinyl, ethylsulfinyl, propylsulfinyl, tert-pentylsulfinyl and octylsulfinyl; an arylsulfinyl group having 6 to 10 carbon atoms such as phenylsulfinyl, tolylsulfinyl and naphthylsulfinyl; a linear chain or branched alkylsulfonyl group having 1 to 18 carbon atoms such as methylsulfonyl, ethylsulfonyl, propylsulfonyl, isopropylsulfonyl, butylsulfonyl and octylsulfonyl; an arylsulfonyl group having 6 to 10 carbon atoms such as phenylsulfonyl, tolylsulfonyl (tosyl), naphthylsulfonyl; an alkyleneoxy groups; a cyano groups; a nitro groups; and halogens such as fluorine, chlorine, bromine and iodine.

Among the iodonium salt-based compounds, the aryl iodonium salt is preferable because of having high stability. Further, it is preferable that the aryl group has a substituent in order to improve fat solubility. Specifically, a linear alkyl group such as methyl, propyl, octyl, decyl, undecyl, dodecyl and tridecyl, a branched alkyl group such as isopropyl, isobutyl, sec-butyl, tert-butyl, isopentyl, neopentyl, tert-pentyl and isohexyl, a functional group in which one or more of H of these hydrocarbon groups is substituted with F, a perfluoroalkyl group and halogen is suitable as the substituent.

The structure of an anion portion of the iodonium salt-based compound is not particularly limited, and examples include those having atoms such as halogen, P, S, B, Al and Ga. Anions having As or Sb can be used, but they are not preferable in dental applications, from the viewpoint of safety. Further, the anion preferably has an organic group such as an alkyl group and/or an alkoxy group and/or an aryl group, and further, most preferably has an organic group such as an alkyl group and/or an alkoxy group and/or an aryl group, in which at least one H is substituted with F. Since the iodonium salt-based compound having such an anion has high solubility with respect to the dental curable composition, it is expected to preventing precipitation during low-temperature storage or long-term storage and to shorten the time for production due to short time dissolving in the composition. Further, an iodonium salt-based compound of an anion having an organic group such as an alkyl group and/or an alkoxy group and/or an aryl group, in which at least one H is substituted with F, can be expected to have higher solubility. When the photoacid generator is precipitated, there is a case where it may cause a decrease in light color stability and a decrease in flexural strength, and therefore it is not preferable. As the anion having an organic group such as an alkyl group and/or an alkoxy group and/or an aryl group, in which at least one H is substituted with F, an anion having any atom can be used. However, from the viewpoint of versatility and safety, those having one or more of P, S, B, Al and Ga are preferable.

Examples of the anion having no alkyl group and/or alkoxy group and/or aryl group include halogens such as chloride and bromide, perhalonic acids such as perchloric acid, aromatic sulfonic acids such as p-toluenesulfonate, camphorsulfonnic acids, nitrates, acetates, chloroacetates, carboxylates, phenolates, tetrafluoroborates, hexafluorophosphates, hexafluoroantimonates, hexafluoroarsenates and the like. Among these, p-toluenesulfonate, camphorsulfonic acid and carboxylate are preferably used.

Since the anionic part of $[A]^-$ of the iodonium salt-based compound of the formula (2) improves the solubility to the dental curable composition, it is preferable that the anion has an organic group such as alkyl group and/or alkoxy group and/or aryl group, in which at least one H is substituted with F. Specifically, the number of carbon atoms of the

alkyl group in the anion part of $[A]^-$ of the iodonium salt-based compound of the formula (2) is 1 to 8, and preferably 1 to 4. Specific examples include a linear alkyl group such as methyl, ethyl, propyl, butyl, pentyl and octyl, a branched alkyl group such as isopropyl, isobutyl, sec-butyl and tert-butyl, and a cycloalkyl group such as cyclopropyl, cyclobutyl, cyclopentyl and cyclohexyl. The ratio (F/H) of the number of hydrogen atoms to fluorine atoms in the alkyl group is 4 or more, and the ratio (F/H) of the number of hydrogen atoms to fluorine atoms in the alkyl group is preferably 9 or more. More preferably, all hydrogen atoms of the hydrocarbon are substituted with fluorine. An iodonium salt consisting of an anion having an alkyl group having a different ratio of a hydrogen atom and a fluorine atom may be compounded in the dental curable composition.

Further, specific examples of the alkyl group include a linear chain or branched perfluoroalkyl group such as $CF_3$, $CF_3CF_2$, $(CF_3)_2CF$, $CF_3CF_2CF_2$, $CF_3CF_2CF_2CF_2$, $(CF_3)_2CFCF_2$, $CF_3CF_2(CF_3)CF$ and $(CF_3)_3C$.

The number of carbon atoms of the alkyl group in the anion part of $[A]^-$ of the iodonium salt-based compound of the formula (2) is 1 to 8, and preferably 1 to 4. Specific examples include a linear alkoxy group such as methoxy, ethoxy, propoxy, butoxy, pentoxy and octoxy, and a branched alkoxy group such as isopropoxy, isobutoxy, sec-butoxy and tert-butoxy. The ratio (F/H) of the number of hydrogen atoms to fluorine atoms in the alkyl group is 4 or more, and the ratio (F/H) of the number of hydrogen atoms to fluorine atoms in the alkyl group is preferably 9 or more. More preferably, all hydrogen atoms of the hydrocarbon are substituted with fluorine. An iodonium salt consisting of an anion having an alkoxy group having a different ratio of a hydrogen atom and a fluorine atom may be compounded in the dental curable composition.

Further, specific examples of the alkoxy group include a linear or branched perfluoroalkoxy group such as $CF_3O$, $CF_3CF_2O$, $CF_3CF_2CF_2O$, $(CF_3)_2CFO$, $CF_3CF_2CF_2CF_2O$, $(CF_3)_2CFCF_2O$, $CF_3CF_2(CF_3)CFO$, $CF_3CF_2CF_2CF_2CF_2O$ and $CF_3CF_2CF_2CF_2CF_2CF_2CF_2CF_2O$.

The phenyl group in the anion part of $[A]^-$ of the iodonium salt compound of the formula (2) may be a phenyl group, in which at least one H is substituted with fluorine atom, an alkyl group and/or an alkoxy group substituted with fluorine atom. The alkyl group and/or alkoxy group substituted with fluorine atom are preferably those described above. Specific examples of particularly preferable phenyl group include perfluorophenyl group such as pentafluorophenyl group ($C_6F_5$), trifluorophenyl group ($C_6H_2F_3$), tetrafluorophenyl group ($C_6HF_4$), trifluoromethylphenyl group ($CF_3C_6H_4$), bis (trifluoromethyl) phenyl group ($(CF_3)_2C_6H_3$), pentafluoroethyl phenyl group ($CF_3CF_2C_6H_4$), bis (pentafluoroethyl) phenyl group ($(CF_3)_2C_6H_3$), trifluoromethyl fluorophenyl group ($CF_3C_6H_3F$), bistrifluoromethyl fluorophenyl group ($(CF_3)_2C_6H_2F$), pentafluoroethyl fluorophenyl group ($CF_3CF_2C_6H_3F$), bispentafluoroethyl fluorophenyl group $(CF_3CF_2)_2C_6H_2F$ and the like. An iodonium salt consisting of an anion having a phenyl group having a different ratio of a hydrogen atom and a fluorine atom may be compounded in the dental curable composition.

As specific examples of the anion portion of $[A]^-$ of the iodonium salt compound of the formula (2), examples of the anion having P include $[(CF_3CF_2)_3PF_3]^-$, $[(CF_3CF_2CF_2)_3PF_3]^-$, $[((CF_3)_2CF)_2PF_4]^-$, $[((CF_3)_2CF)_3PF_3]^-$, $[((CF_3)_2CF)_4PF_2]^-$, $[((CF_3)_2CFCF_2)_2PF_4]^-$, $[((CF_3)_2CFCF_2)_3PF_3]^-$ and the like. Examples of the anion having S include $[(CF_3SO_2)_3C]^-$, $[(CF_3CF_2SO_2)_3C]^-$, $[(CF_3CF_2CF_2SO_2)_3C]^-$, $[(CF_3CF_2CF_2CF_2SO_2)_3C]^-$, $[CF_3CF_2CF_2CF_2SO_3]^-$, $[CF_3CF_2CF_2SO_3]^-$, $[(CF_3CF_2SO_2)_3C]^-$, $[(SO_2CF_3)_3N]^-$, $[(SO_2CF_2CF_3)_2N]^-$, $[((CF_3)C_6H_4)SO_3]^-$, $[SO_3(CF_2CF_2CF_2CF_2)SO_3]^{2-}$ and the like. Examples of the anion having B include $[B(C_6F_5)4]^-$, $[(C_6H_5)B((CF_3)_2C_6H_3)_3]^-$, $[(C_6H_5)B(C_6F_5)_3]^-$ and the like. Examples of an anion having Ga include $[((C_6F_5)_3(C_6H_5)Ga)]^-$, $[((C_6F_5)_3(C_4F_9)Ga)]^-$, $[((C_6H_2F_3)_4Ga)]^-$, $[((CF_3)_2C_6H_3)_4Ga]^-$, $[[((CF_3)_4Ga)]^-$, $[Ga(C_6F_5)_4]^-$ and the like. Examples of anions having Al include $[((CF_3)_3CO)_4Al]^-$, $[((CF_3CF_2)_3CO)_4Al]^-$.

The photoacid generator that can be used in the dental curable composition of the present invention is not limited to the photoacid generator described in the specific example, and two or more types can be used in combination.

[(B-3) Photopolymerization Accelerator]

The (B-3) photopolymerization accelerator which is used for the dental curable composition of the present invention is not particularly limited as long as it has polymerization promoting ability, and any known photopolymerization accelerator commonly used in the dental field may be used without any limitation. As the (B-3) photopolymerization accelerator, a primary to tertiary amine compound such as an aromatic amine compound and an aliphatic amine compound, a phosphine compound, an organic metal compound, a transition metal compound of the group 4 in the periodic table, a thiourea derivative, a sulfinic acid and a salt thereof, a borate compound, a sulfur-containing reductive inorganic compound, a nitrogen-containing reductive inorganic compound, a barbituric acid derivative, a triazine compound, a halogen compound and the like can be used.

Aromatic amine compound refers to a compound in which one or more H of ammonia ($NH_3$) is replaced with an aromatic ring. Aromatic amine compound in which one H of $NH_3$ is substituted with an aromatic ring is classified into an aromatic primary amine compound, aromatic amine compound in which one H of $NH_3$ is substituted with an aromatic ring and one H of remaining two H is substituted with an aromatic ring or an alkyl group is classified into an aromatic secondary amine compound, and aromatic amine compound in which one H of $NH_3$ is substituted with an aromatic ring

and remaining two H are substituted with an aromatic ring or an alkyl group is classified into an aromatic tertiary amine compound.

Specific examples of the aromatic primary amine compound include aniline. Specific examples of the aromatic secondary amine compound include N-protected amino acid (ester) such as N-phenyl benzylamine, N-benzyl-p-anisidine, N-benzyl-o-phenetidine, N-phenylglycine ethyl and N-phenylglycine. Specific examples of the aromatic tertiary amine compound include N,N-dimethylaniline, N,N-diethylaniline, N,N-di-n-butylaniline, N,N-dibenzylaniline, p-N,N-dimethyl-toluidine, m-N,N-dimethyl-toluidine, p-N,N-diethyl-toluidine, p-bromo-N,N-dimethylaniline, m-chloro-N,N-dimethylaniline, p-dimethylamino benzaldehyde, p-dimethylamino acetophenone, p-dimethylamino benzoic acid, p-dimethylamino benzoic acid ethyl ester, p-dimethylamino benzoic acid isoamyl estel, p-dimethylamino benzoic acid 2-butoxyethyl, p-dimethylamino benzoic acid 2-ethylhexyl, p-dimethylamino benzoic acid amino ester, N,N-dimethyl anthranic acid methyl ester, N,N-dihydroxyethyl aniline, N,N-diisopropanol aniline, p-N,N-dihydroxyethyl-toluidine, p-N,N-dihydroxypropyl-toluidine, p-dimethylamino phenyl alcohol, p-dimethylamino styrene, N,N-dimethyl-3,5-xylidine, 4-dimethylamino pyridine, N,N-dimethyl-$\alpha$-naphthylamine, N.N-dimethyl-$\beta$-naphthylamine and the like. Among them, p-dimethylamino benzoic acid ethyl ester is preferable.

The phosphine compound refers to a compound which is trisubstituted on P atom with organic groups, and the aromatic phosphine compound refers to a compound which is substituted on P atom with a phenyl group which may have one or more substituents. Specific examples of the phosphine compound include trimethylphosphine, tributylphosphine, trihexylphosphine, tri-n-octylphosphine, tricyclohexylphosphine, tri (2-thienyl) phosphine, diphenylpropyl phosphine, di-tert-butyl (3-methyl-2-butenyl) phosphine, methyldiphenyl phosphine, triphenyl phosphine, 2-(diphenylphosphino) styrene, 3-(diphenylphosphino) styrene, 4-(diphenylphosphino) styrene, allyldiphenyl phosphine, 2-(diphenylphosphino) benzaldehyde, 3-(diphenylphosphino) benzaldehyde, 4-(diphenylphosphino) benzaldehyde, 2-(phenylphosphine) benzoic acid, 3-(phenylphosphino) benzoic acid, 4-(phenylphosphino) benzoic acid, tris (2-methoxyphenyl) phosphine, tris (3-methoxyphenyl) phosphine, tris (4-methoxyphenyl) phosphine, 2-(diphenylphosphino) biphenyl, tris (4-fluorophenyl) phosphine, tri (o-trill) phosphine, tri (m-trill) phosphine, tri (p-trill) phosphine, 2-(dimethylamino) phenyldiphenyl phosphine, 3-(dimethylamino) phenyldiphenyl phosphine, 4-(dimethylamino) phenyldiphenyl phosphine, 2,2'-bis (diphenylphosphino) biphenyl, bis [2-(diphenylphosphino) phenyl] ether and the like. Among them, triphenylphosphine, 4-(phenylphosphino) benzoic acid, tri (o-tolyl) phosphine, tri (m-tolyl) phosphine and tri (p-tolyl) phosphine are preferable.

Aliphatic amine compounds refer to compounds in which one or more H of ammonia ($NH_3$) are substituted with alkyl group. As for the alkyl group, $CH_3$- and $CH_2$- are classified as a primary alkyl group, the one in which one H of -$CH_2$- is substituted with a substituent is classified as a secondary alkyl group, and the one in which two H of -$CH_2$- are substituted with substituents is classified as a tertiary alkyl group. Aliphatic amine in which one H of $NH_3$ is substituted with an alkyl group is classified into an aliphatic primary amine compound, aliphatic amine compound in which two H of $NH_3$ are substituted with an alkyl group is classified into an aliphatic secondary amine compound, and aliphatic amine compound in which three H of $NH_3$ are substituted with an alkyl group is classified into an aliphatic tertiary amine compound.

Specific examples of the aliphatic primary amine compound include amino acid or amino acid ester such as benzhydrylamine, triphenylmethylamine and glycine. Specific examples of the aliphatic secondary amine compound include dibenzylamine, N-benzyl-1-phenylethylamine, bis (1-phenylethyl) amine, bis (4-cyanobenzyl) amine, N-benzyl protected amino acid and N-benzyl protected amino acid ester. Specific examples of the aliphatic tertiary amine compound include tributylamine, tripropylamine, triethylamine, N,N-dimethyl hexylamine, N,N-dimethyl dodecylamine, N,N-dimethyl stearylamine, N-[3-(dimethylamino) propyl] acrylamide, N,N-dimethyl formamide dimethylacetal, N,N-dimethylacetamide dimethylacetal, N,N-dimethylformamide diethylacetal, N,N-dimethylformamide dipropylacetal, N,N-dimethylformamide di-tert-butylacetal, 1-(2-hydroxyethyl) ethyleneimine, N,N-dimethyl ethanolamine, N,N-dimethyl isopropanolamine, N,N-diisopropyl ethanolamine, N-methyl diethanolamine, N-ethyl diethanolamine, N-ethyl diethanolamine, N-butyl diethanolamine, N-lauryl diethanolamine, N-stearyl diethanolamine, triethanolamine, triisopropanolamine, tribenzylamine, dibenzylglycine ethylester, N'-(2-hydroxyethyl)-N,N,N'-trimethylethylene diamine, 2-(dimethylamino)-2-methyl-1-propanol, N,N-dimethyl-2,3-dihydroxy propylamine, N,N-diethylethanolamine, 1-methyl-3-pyrrolidinol, 1-(2-hydroxyethyl) pyrrolidine, 1-isopropyl-3-pyrrolidinol, 1-piperidin ethanol, 2-[2-(dimethylamino) ethoxy] ethanol, N,N-dimethylglycine, N,N-dimethylglycine methyl, N,N-diethylglycine methyl, N,N-dimethylglycine ethyl, N,N-diethylglycine sodium, 2-(dimethylamino) ethylacetate, N-methylimimino diacetic acid, N,N-dimethylamino ethylacrylate, N,N-diethylamino ethylmethacrylate, N,N-diisopropylamino ethylmethacrylate, N,N-dibutylamino ethylmethacrylate, N,N-dibenzylamino ethylmethacrylate, 3-dimethylamino propionitrile, tris (2-cyanoethyl) amine, N,N-dimethyl allylamine, N,N-diethyl allylamine and triallylamine.

Specific examples of the above organic metal compound include an organic metal compound containing scandium (Sc), titanium (Ti), vanadium (V), manganese (Mn), iron (Fe), cobalt (Co), nickel (Ni), copper (Cu), tin (Sn), zinc (Zn) and/or zirconia (Zr), and an organic metal compound containing tin (Sn), vanadium (V) and/or copper (Cu) is preferable. Specific examples of the organic metal compound containing tin (Sn) include dibutyl-tin-diacetate, dibutyl-tin-dimaleate, dioctyl-tin-dimaleate, dioctyl-tin-dilaurate, dibutyl-tin-dilaurate, dioctyl-tin- dinonanoate, dioctyl-tin-S,S'-bis-isooctyl mercapto acetate, tetramethyl-1,3-diacetoxy distanoxane and the like. Specific examples of the organic metal compound containing vanadium (V) include acetylacetone vanadium, divanadium tetraoxide, vanadyl acetylacetonate, vanadyl stearate oxide, vanadyl oxalate, vanadyl sulphate, oxobis (1-phenyl-1,3-butandionate) vanadium, bis (maltlate) oxovanadium, vanadium pentoxide and sodium metavanadate. Specific examples of the organic metal compound containing copper (Cu) include copper acetylacetone, copper naphthenate, copper octylate, copper stearate and copper acetate.

Among these, a trivalent or tetravalent vanadium compound and a divalent copper compound are preferable. Among them, because of having higher polymerization accelerating ability, a trivalent or tetravalent vanadium compound is more preferable, and a tetravalent vanadium compound is most preferable. A plurality of kinds of these transition metal compounds in the period 4 in the periodic table may be used in combination, if necessary. The compounding amount of transition metal compound is preferably 0.0001 to 1 parts by mass with respect to 100 parts by mass of the total amount of the (A) polymerizable monomer. When the compounding amount is less than 0.0001 parts by mass, there is a case where the polymerization accelerating effect is insufficient, and when the compounding amount exceeds 1 part by mass, there is a case where it causes discoloration or gelation of the dental curable composition and the storage stability is lowered.

Any known thiourea derivatives can be used as the thiourea derivative without any limitation. Specific examples of the thiourea derivatives include dimethylthiourea, diethylthiourea, tetramethylthiourea, (2-pyridyl) thiourea, N-methylthiourea, ethylenethiourea, N-allylthiourea, N-allyl-N'-(2-hydroxyethyl) thiourea, N-benzylthiourea, 1,3-dicyclohexyl thiourea, N,N'-diphenylthiourea, 1,3-di (p-tolyl) thiourea, 1-methyl-3-phenylthiourea, N-acetylthiourea, N-benzoylthiourea, diphenylthiourea, dicyclohexylthiourea and the like. Among these, (2-pyridyl) thiourea, N-acetylthiourea and N-benzoylthiourea are preferable. A plurality of kinds of these thiourea derivatives can be used in combination, if necessary. The compounding amount of the thiourea derivative is preferably 0.1 to 5 parts by mass with respect to 100 parts by mass of the total amount of the (A) polymerizable monomers. When the compounding amount is less than 0.1 parts by mass, there is a case where the ability as a polymerization accelerator is insufficient, and when the compounding amount exceeds 5 parts by mass, the storage stability may be lowered.

Examples of the sulfinic acid and its salt include p-toluene sulfinic acid, sodium p-toluene sulfinate, potassium p-toluene sulfinate, lithium p-toluene sulfinate, calcium p-toluene sulfinate, benzenesulfinic acid, sodium benzene sulfinate, potassium benzene sulfinate, lithium benzenesulfinate, calcium benzenesulfinate, 2,4,6-trimethyl benzenesulfinic acid, sodium 2,4,6-trimethyl benzenesulfinate, potassium 2,4,6-trimethyl benzenesulfinate, lithium 2,4,6-trimethyl benzenesulfinate, calcium 2,4,6-trimethyl benzenesulfinate, 2,4,6-triethyl benzenesulfinic acid, sodium 2,4,6-triethyl benzenesulfinate, potassium 2,4,6-triethyl benzenesulfinate, lithium 2,4,6-triethyl benzenesulfinate, calcium 2,4,6-triethyl benzenesulfinate, 2,4,6-triisopropyl benzenesulfinic acid, sodium 2,4,6-triisopropyl benzenesulfinate, potassium 2,4,6-triisopropyl benzenesulfinate, lithium 2,4,6-triisopropyl benzenesulfinate, calcium 2,4,6-triisopropyl benzenesulfinate and the like. Among them, sodium benzenesulfinate, sodium p-toluenesulfinate, and sodium 2,4,6-triisopropyl benzenesulfinate are particularly preferable.

As the borate compound, specific examples of the borate compound having one aryl group in one molecule include trialkylphenylboron, trialkyl (p-chlorophenyl) boron, trialkyl (p-fluorophenyl) boron, trialkyl (3,5-bistrifluoro methyl) phenyl boron, trialkyl [3,5-bis (1,1,1,3,3,3-hexafluoro-2-methoxy-2-propyl) phenyl] boron, trialkyl (p-nitrophenyl) boron, trialkyl (m-nitrophenyl) boron, trialkyl (p-butylphenyl) boron, trialkyl (m-butylphenyl) boron, trialkyl (p-butyloxyphenyl) boron, trialkyl (m-butyloxyphenyl) boron, trialkyl (p-octyloxyphenyl) boron and trialkyl (m-octyloxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having two aryl groups in one molecule include dialkyl diphenylboron, dialkyl di (p-chlorophenyl) boron, dialkyl di (p-fluorophenyl) boron, dialkyl di (3,5-bistrifluoro methyl) phenyl boron, dialkyl di [3,5-bis (1,1,1,3,3,3-hexafluoro-2-methoxy-2-propyl) phenyl] boron, dialkyl di (p-nitrophenyl) boron, dialkyl di (m-nitrophenyl) boron, dialkyl di (p-butylphenyl) boron, dialkyl di (m-butylphenyl) boron, dialkyl di (p-butyl oxyphenyl) boron, dialkyl di (m-butyl oxyphenyl) boron, dialkyl di (p-octyl oxyphenyl) boron and dialkyl di (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having three aryl groups in one molecule include monoalkyl triphenylboron, monoalkyl tri (p-chlorophenyl) boron, monoalkyl tri (p-fluorophenyl) boron, monoalkyl tri (3,5-bistrifluoro methyl) phenyl boron, monoalkyl tri [3,5-bis (1,1,1,3,3,3-hexafluoro-2-methoxy-2-propyl) phenyl] boron, monoalkyl tri (p-nitrophenyl) boron, monoalkyl tri (m-nitrophenyl) boron, monoalkyl tri (p-butylphenyl) boron, monoalkyl tri (m-butylphenyl) boron, monoalkyl tri (p-butyl oxyphenyl) boron, monoalkyl tri (m-butyl oxyphenyl) boron, monoalkyl tri (p-octyl oxyphenyl) boron and monoalkyl tri (m-octyl oxyphenyl) boron (the alkyl group is at least one selected from the group consisting of n-butyl

group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having four aryl groups in one molecule include tetraphenylboron, tetra kis (p-chlorophenyl) boron, tetra kis (p-fluorophenyl) boron, tetra kis (3,5-bistrifluoro methyl) phenyl boron, tetra kis [3,5-bis (1,1,1,3,3,3-hexafluoro-2-methoxy-2-propyl) phenyl] boron, tetra kis (p-nitrophenyl) boron, tetra kis (m-nitrophenyl) boron, tetra kis (p-butylphenyl) boron, tetra kis (m-butylphenyl) boron, tetra kis (p-butyl oxyphenyl) boron, tetra kis (m-butyl oxyphenyl) boron, tetra kis (p-octyl oxyphenyl) boron, tetra kis (m-octyl oxyphenyl) boron, (p-fluorophenyl) triphenylboron, (3,5-bis trifluoromethyl) phenyl triphenylboron, (p-nitrophenyl) triphenylboron, (m-butyl oxyphenyl) triphenylboron, (p-butyl oxyphenyl) triphenylboron, (m-octyl oxyphenyl) triphenylboron and (p-octyl oxyphenyl) triphenylboron, and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like).

Among these aryl borate compounds, it is more preferable to use a borate compound having 3 or 4 aryl groups in one molecule from the viewpoint of storage stability. Further, these aryl borate compounds can be used alone or as a mixture of two or more.

Examples of the sulfur-containing reductive inorganic compound include sulfites, bisulfites, pyrosulfites, thiosulfates, thionates and dithionite. Specific examples include sodium sulfite, potassium sulfite, calcium sulfite, ammonium sulfite, sodium bisulfite, potassium bisulfite, 3-mercaptopropyl trimethoxysilane, 2-mercaptobenzoxazole, decanethiol, thiobenzoic acid and the like.

Examples of the nitrogen-containing reductive inorganic compound include nitrites, and specific examples include sodium nitrite, potassium nitrite, calcium nitrite, ammonium nitrite and the like.

Specific examples of the barbituric acid derivative include salts (alkali metals or alkaline earth metals are preferred) of barbituric acid, 1,3-dimethyl barbituric acid, 1,3-diphenyl barbituric acid, 1,5-dimethyl barbituric acid, 5-butyl barbituric acid, 5-ethyl barbituric acid, 5-isopropyl barbituric acid, 5-cyclohexyl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl barbituric acid, 1,3-dimethyl-5-cyclopentyl barbituric acid, 1,3-dimethyl-5-cyclohexyl barbituric acid 1,3-dimethyl-5-phenyl barbituric acid, 1-cyclohexyl-1-ethyl barbituric acid, 1-benzyl-5-phenyl barbituric acid, 5-methyl barbituric acid, 5-propyl barbituric acid, 1,5-diethyl barbituric acid, 1-ethyl-5-methyl barbituric acid, 1-ethyl-5-isobutyl barbituric acid, 1,3-diethyl-5-butyl barbituric acid, 1-cyclohexyl-5-methyl barbituric acid, 1-cyclohexyl-5-ethyl barbituric acid, 1-cyclohexyl-5-octyl barbituric acid, 1-cyclohexyl-5-hexyl barbituric acid, 5-butyl-1-cyclohexyl barbituric acid, 1-benzyl-5-phenyl barbituric acid and thiobarbituric acids. Specifically, the salts of these barbituric acids include sodium 5-butyl barbiturate, sodium1,3,5-trimethyl barbiturate, sodium 1-cyclohexyl-5-ethyl barbiturate and the like.

Specific examples of the halogen compound include dilauryl dimethyl ammonium chloride, lauryl dimethyl benzyl ammonium chloride, benzyl trimethyl ammonium chloride, tetramethyl ammonium chloride, benzyl dimethyl acetyl ammonium chloride, dilauryl dimethyl ammonium bromide and the like.

There is no problem even if these (B-1) photosensitizers, (B-2) photoacid generators and (B-3) photopolymerization accelerators, which are polymerization initiators, are subjected to a secondary treatment such as finely pulverization, adsorption on a carrier and encapsulation in a microcapsule, if necessary. Furthermore, these photopolymerization initiators can be used not only singly but also in combinations of two or more, regardless of the polymerization manner or the polymerization method.

The compounding amount of the (B) polymerization initiator is preferably 0.5 to 10 parts by mass with respect to 100 parts by mass of the total amount of the (A) polymerizable monomer. When the compounding amount is less than 0.5 parts by mass, there is a case where the mechanical strength is insufficient. When the compounding amount is more than 10 parts by mass, although it has sufficient curability, the sensitivity to light is shortened, and discoloration such as browning of the cured body may increase, and therefore it is not preferable.

Among the (B) polymerization initiators, it is preferable to contain a combination of (B-1) photosensitizer, (B-2) photoacid generator and (B-3) polymerization accelerator as the (B) polymerization initiator. In this case, it is preferable to contain a diaryliodonium salt compound as the (B-2) photoacid generator. It is also preferable to contain a tertiary amine compound as the (B-3) polymerization accelerator. By containing a diaryliodonium salt compound as the (B-2) photoacid generator and a tertiary amine compound as the (B-3) polymerization accelerator, it is possible to exhibit excellent photocuring property.

It is more preferable that the dental curable composition does not substantially comprise an aromatic amine compound as the (B-3) polymerization accelerator. In the present specification, the phrase "not substantially comprise" means that it is not intentionally compounded. For example, in the case that an aromatic amine is contained as impurity in a component contained in a dental curable composition and in the case that an aromatic amine is contained only in a trace amount of less than 0.1 parts by mass with respect to 100 parts by mass of the total amount of the (A) polymerizable monomer contained in the dental curable composition, when the properties of the dental curable composition are not

affected, these correspond to the "not substantially comprise". When an aromatic amine compound is used as a polymerization accelerator, there is a tendency that the photopolymerization-accelerating property is higher than the case of using an aliphatic amine compound, but there is a problem that the sunlight stability is significantly reduced. Therefore, when an aromatic amine compound is used, it is required to compound a large amount of an ultraviolet absorber in order to ensure sunlight stability, thus the possibility of reducing fluorescence and mechanical property arises. When the dental curable composition of the present invention contains an amine compound as the (B-3) polymerization accelerator, an aliphatic amine compound is preferable.

[(C) fluorescent agent]

The dental curable composition of the present invention contains (C) fluorescent agent. As the (C) fluorescent agent, a known fluorescent agent commonly used can be used without any limitation.

Specific examples of the (C) fluorescent agent that can be used in the present invention include a terephthalic acid ester compound such as dimethyl-2,5-dihydroxy terephthalate, diethyl-2,5-dihydroxy terephthalate, dimethyl-2,5-diamino terephthalate, diethyl-2,5-diamino terephthalate, dimethylamino terephthalate, and diethylamino terephthalate; a benzoxazole compound such as 2,5-thiophenediylbis (5-tert-butyl-1,3-benzoxazole and 2,2'-(1,2-ethylenediyldi-4,1-phenylene) bisbenzoxazole; and phthalic acid derivatives such as o-phthalaldehyde.

Among these fluorescent agents, the terephthalic acid ester compound represented by formula (3) is preferable because it is relatively easily available, does not affect the color tone when compounded into a dental curable composition, and emits fluorescence similar to that of a natural tooth. In particular, it is more preferable to use diethyl-2,5-dihydroxy terephthalate, which corresponds to the structure represented by formula (3). These fluorescent agents may be used singly or as a mixture of two or more. It is possible to contain only the terephthalic acid ester compound represented by formula (3) as the fluorescent agent and to contain only diethyl-2,5-dihydroxy terephthalate as the fluorescent agent.

[Formula (3)]

[Chemical formula 4]

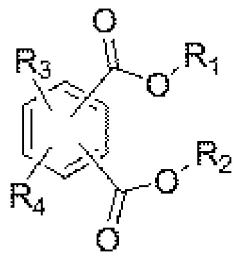

(In the formula, $R_1$ and $R_2$ each independently represent an alkyl group, $R_3$ represents a hydrogen atom, an amino group or a hydroxyl group, and $R_4$ represents an amino group or a hydroxyl group. The alkyl group has 1 to 3 carbon atoms, such as a methyl group, an ethyl group, an n-propyl group, or an i-propyl group.)

The compounding amount of the (C) fluorescent agent in the dental curable composition of the present invention is preferably 0.001 to 0.1 part by mass or more, more preferably 0.005 to 0.1 part by mass or more, with respect to 100 parts by mass of the total amount of the (A) polymerizable monomer. When the compounding amount of the fluorescent agent is less than 0.001 parts by mass, there is a case where the fluorescence is poor. When the compounding amount of the fluorescent agent is more than 0.1 parts by mass, there is a case where the fluorescence is too strong compared to a natural tooth and appear unnatural.

[(D) Ultraviolet absorber]

The dental curable composition of the present invention include an ultraviolet absorber.

In the present invention, the dental curable composition comprises (D-1) compound having at least one first absorption maximum in a wavelength region of 250 to 320 nm and at least one second absorption maximum in a wavelength region of 320 to 400 nm, wherein a ratio of an absorbance of the first absorption maximum to an absorbance of the second absorption maximum is within the range of 1 to 4 as the (D) ultraviolet absorber. The compounds not

having at least one first absorption maximum in a wavelength region of 250 to 320 nm are not contained in the compound corresponding to the (D-1) compound. Moreover, the compounds not having at least one second absorption maximum in a wavelength region of 320 to 400 nm are not contained in the compound corresponding to the (D-1) compound. The compound corresponding to the (D-1) compound can be selected from known ultraviolet absorbers that are commonly used.

The dental curable composition of the present invention may comprises only (D-1) compound having at least one first absorption maximum in a wavelength region of 250 to 320 nm and at least one second absorption maximum in a wavelength region of 320 to 400 nm, wherein a ratio of an absorbance of the first absorption maximum to an absorbance of the second absorption maximum is within the range of 1 to 4 as the (D) ultraviolet absorber. The dental curable composition of the present invention may not contain a compound that does not correspond to the (D-1) compound as the (D) ultraviolet absorber. As the (D) ultraviolet absorber consisting of a compound not corresponding to the (D-1) compound, a commonly used known ultraviolet absorber can be used.

Furthermore, the dental curable composition of the present invention may contain only one kind of the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the range of 1 to 4 as the (D) ultraviolet absorber.

Furthermore, the dental curable composition of the present invention may contain two or more kinds of the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the range of 1 to 4 as the (D) ultraviolet absorber.

Many of the fluorescent agents compounded in a dental curable composition absorb near-ultraviolet light of 320 to 400 nm and emit fluorescence. Furthermore, some polymerization initiators compounded in a dental curable composition have absorption in the near ultraviolet region of 320 to 400 nm. In the investigation of the present inventors, it has been found that when a having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the range of 1 to 4 is used as ultraviolet absorber, fluorescence, mechanical property and sunlight stability are excellent, and the present invention is completed. In the present invention, the absorption maximum refers to a point where the absorbance curve is upwardly convex and the derivative of the absorbance curve is zero, in the absorption spectrum of ultraviolet absorber. In the present invention, the first absorption maximum is an absorption maximum present in the wavelength region of 250 to 320 nm, and the second absorption maximum is an absorption maximum present in the wavelength region of 320 to 400 nm. The compound (D-1) contained as the (D) ultraviolet absorber in the present invention has at least one first absorption maximum and at least one second absorption maximum. The compound (D-1) may have multiple first absorption maximum and multiple second absorption maximum, respectively.

In the compound corresponding to (D-1) compound contained as the (D) ultraviolet absorber in the present invention, the ratio of an absorbance of the first absorption maximum to an absorbance of the second absorption maximum is within the range of 1 to 4, preferably 1.1 to 3, and more preferably 1.2 to 2. When a compound having the ratio of an absorbance of less than 1 is used as the (D) ultraviolet absorber, the absorption of the fluorescent agent and the polymerization initiator in the near ultraviolet region is inhibited, and the fluorescence and mechanical property of the curable composition tend to decrease. In addition, the distribution of ultraviolet absorption in the wavelength region of 250 to 400 nm becomes biased, and therefore the sunlight stability also decreases. On the other hand, when a compound having the ratio of an absorbance of more than 4 is used as the (D) ultraviolet absorber, excellent fluorescence is exhibited, but the distribution of ultraviolet absorption in the wavelength region of 250 to 400 nm is biased, and therefore the sunlight stability decreases.

It is preferable that the compound corresponding to the (D-1) compound contained as the (D) ultraviolet absorber used in the present invention has a second absorption maximum wavelength of less than 350 nm. When the compound corresponding to the (D-1) compound has a second absorption maximum wavelength of less than 350 nm, particularly excellent fluorescence, mechanical property and sunlight stability are exhibited. When the second absorption maximum wavelength is present in 350 nm or more, there is a case where because the overlap with the ultraviolet absorption region of the fluorescent agent and the polymerization initiator becomes large, the fluorescence and mechanical property may be slightly deteriorated.

The absorbance at the maximum absorption wavelength of the ultraviolet absorber containing the compound corresponding to the (D-1) compound can be determined by dissolving the ultraviolet absorber in an organic solvent in which the ultraviolet absorber is soluble and measuring the absorbance with a spectrophotometer. The organic solvent is not particularly limited as long as each compound is soluble in the organic solvent. However, it is necessary to select an organic solvent having a transmittance of 50% or more in the ultraviolet region of 250 to 400 nm so as not to inhibit

the absorbance in the ultraviolet region of the ultraviolet absorber containing the compound corresponding to the (D-1) compound. Examples of the organic solvent having a transmittance of 50% or more in the ultraviolet region of 250 to 400 nm include acetonitrile, n-hexane, methanol, ethanol, and the like. Among these, any solvent in which the ultraviolet absorber containing the compound corresponding to the (D-1) compound is soluble can be selected. Among these organic solvents, acetonitrile is particularly preferable because of its high transmittance in the ultraviolet region of 250 to 400 nm.

For measuring the absorbance of a compound corresponding to the (D-1) compound, a cell that does not absorb in the ultraviolet region of 250 to 400 nm is used. Generally, a quartz cell is used as such a cell.

The concentration of the ultraviolet absorber containing the compound corresponding to the (D-1) compound in the organic solvent may be appropriately adjusted so that the absorbance of both the first absorption maximum and the second absorption maximum are within the range of 0.2 to 2. When the absorbance is not within this range, transmittance is not within the effective range, therefore measurement error becomes large. In addition, since the absorption coefficient differs depending on the type of ultraviolet absorber, the concentration in the organic solvent when adjusted so that the absorbance is within the range of 0.2 to 2 differs for each ultraviolet absorber. However, the ratio of an absorbance of the first absorption maximum to an absorbance of the second absorption maximum, which is derived from the absorbance measurement results of the ultraviolet absorber (including the compound corresponding to the (D-1) compound) in the present invention, is not affected by the concentration of the ultraviolet absorber in the organic solvent, so long as the absorbance is appropriately adjusted to be in the range of 0.2 to 2.

The ratio of an absorbance of the first absorption maximum to an absorbance of the second absorption maximum is calculated by dividing the absorbance of the peak top of the first absorption maximum existing in the wavelength region of 250 to 320 nm by the absorbance of the peak top of the second absorption maximum existing in the wavelength region of 320 to 400 nm. When multiple absorption maxima exist in the wavelength region of 250 to 320 nm and/or the wavelength region of 320 to 400 nm, the total absorbance of the peak tops of all the absorption maxima existing in each wavelength region is used.

Specific examples of the compound corresponding to the (D-1) compound that can be used in the present invention include benzotriazole compounds such as 2-(2H-benzotriazol-2-yl)-p-cresol, 2-(2-hydroxy-5-tert-octylphenyl)-benzotriazole, 2- [2-hydroxy-5 - [2-(methacryloyloxy)-ethyl] phenyl]-2H-benzotriazole and 2-(5-chloro-2-benzotriazolyl)-6-tert-butyl-p-cresol; benzophenone compounds such as 2-hydroxy-4-methoxybenzophenone, 2,2'-dihydroxy-4,4'-dimethoxybenzophenone and 2-hydroxy-4-n-octyloxybenzophenone; and triazine compounds such as 2-(4,6-diphenyl-1,3,5-triazin-2-yl)-5-[2-(2-ethylhexanoyloxy)ethoxy] phenol.

Among these compounds, benzophenone compounds are preferable because they have excellent sunlight stability, fluorescence, and mechanical property, as well as excellent solubility with respect to a polymerizable monomer. From the results of the investigation by the present inventors, it has been found that a benzophenone compound are more excellent in solubility with respect to a polymerizable monomer than a triazine compound. Also, it has been found that there is a tendency that a benzophenone compound exhibits better mechanical property and higher fluorescence than a benzotriazole compound.

Among the benzophenone compounds, a benzophenone compound having a hydroxy group is more preferable, and furthermore, a benzophenone compound represented by formula (1) is particularly preferable because they have excellent solubility with respect to a polymerizable monomer, fluorescence, mechanical property and sunlight stability.

[Formula (1)]

[Chemical formula 5]

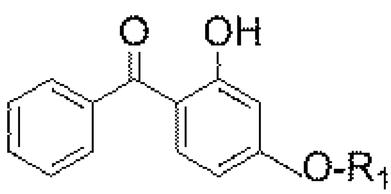

(In the formula (1), $R_1$ is an organic group having 2 to 10 carbon atoms.)

As $R_1$ in the benzophenone compound represented by formula (1), any organic group having 2 to 10 carbon atoms can be used without any problems. More specifically, examples of the structure of $R_1$ include linear alkyl groups having 2 to 10 carbon atoms.

Specific examples of the benzophenone compound represented by formula (1) include 2-hydroxy-4-methoxybenzophenone, 2-hydroxy-4-n-octyloxybenzophenone and the like. Among these, 2-hydroxy-4-n-octyloxybenzophenone is particularly preferable due to its high solubility.

When an aromatic amine compound is used as the polymerization accelerator, it is required to compound a large amount of an ultraviolet absorber in order to ensure the sunlight stability. However, the compound represented by chemical formula (1) and corresponding to the (D-1) compound has excellent solubility with respect to a polymerizable monomer and therefore can be compounded in a large amount. Therefore, even when an aromatic amine compound is used as the polymerization accelerator, it is possible to sufficiently reduce the sunlight stability. However, when an excessive amount of the compound corresponding to the (D-1) compound is compounded as the ultraviolet absorber, there is a possibility that the fluorescence and mechanical property is deteriorated. Thus, in a particularly preferable embodiment of the present invention, it is preferable that the composition does not substantially comprise an aromatic amine compound as the polymerization accelerator. In particular, by using, as the (B) polymerization initiator, (B-1) photosensitizer, (B-2) photoacid generator containing a diaryliodonium salt compound and (B-3) polymerization accelerator containing a tertiary amine compound, sufficiently high mechanical property is exhibited even when an aromatic amine compound is not contained, therefore it is possible to reduce sunlight stability without compounding a large amount of the compound corresponding to the (D-1) compound as the ultraviolet absorber.

Accordingly, the compound corresponding to the (D-1) compound as the (D) ultraviolet absorber in the present invention is excellent in sunlight stability, fluorescence, and mechanical property, and has excellent in solubility with respect to a polymerizable monomer, and therefore has the effects of being excellent in versatility in producing a dental curable composition, shortening the time for production, and reducing the occurrence of uneven coloring of the curable composition.

The compounding amount of the compound corresponding to the (D-1) compound in the dental curable composition of the present invention is preferably 0.01 to 2 parts by mass or more, more preferably 0.1 to 1 parts by mass or more, with respect to 100 parts by mass of the total amount of the (A) polymerizable monomer. When the compounding amount of the ultraviolet absorption is less than 0.01 parts by mass, the sunlight stability may be insufficient and discoloration may occur due to exposure to ultraviolet light. When the compounding amount of the ultraviolet absorption is more than 2 parts by mass, sufficient sunlight stability is exhibited, but mechanical property may decrease.

The compound corresponding to the (D-1) compound contained as the (D) ultraviolet absorber in the present invention exhibits excellent fluorescence, sunlight stability and mechanical property even when compounded alone in the dental curable composition. Therefore, the dental curable composition of the present invention may not contain a compound that does not correspond to the (D-1) compound as the ultraviolet absorber. In the case of achieving to have at least one first absorption maximum in the wavelength region of 250 to 320 nm and at least one second absorption maximum in the wavelength region of 320 to 400 nm and the ratio of an absorbance of the first absorption maximum to an absorbance of the second absorption maximum is within the range of 1 to 4 by adjusting the dental curable composition by compounding a combination of a plurality of compounds not corresponding to the (D-1) compound, the solubilities of an ultraviolet absorber, a polymerization initiator, a fluorescent agent and an additive such as other polymerization inhibitor with respect to a polymerizable monomer decreases, therefore these components precipitate after long-term storage, and therefore the effect of the present invention is not exhibited sufficiently.

[(E) Filler]

The dental curable composition of the present invention may contain (E) filler. As the (E) filler used in the present invention, a known filler commonly used can be used without any limitation.

The type of the (E) filler is not limited as long as it is a known filler, and a filler suitable for the application can be compounded, and it is preferable that a filler such as an inorganic filler, an organic filler, an organic-inorganic composite filler and an ion sustained release glass is compounded. In the dental curable composition of the present invention, the filler described in the specific example may be used alone, or two or more kinds of fillers may be used in combination.

As the inorganic filler, the chemical composition is not particularly limited, but specific examples include silicon dioxide, alumina, titania, silica-titania, silica-titania-barium oxide, silica-zirconia, silica-alumina, lanthanum glass, borosilicate glass, soda glass, barium glass, strontium glass, glass ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass and the like. Particularly, barium fluoroaluminosilicate glass, strontium fluoroaluminosilicate glass, fluoroaluminosilicate glass and the like, which are used in dental glass ionomer cement, resin reinforced glass ionomer cement and resin cement and the like, can also be suitably used. The fluoroaluminosilicate glass as used herein has a basic structure of silicon oxide and aluminum oxide and contains an alkali metal for introducing non-crosslinked oxygen. The fluoroaluminosilicate glass further has an alkaline earth metal including strontium and fluorine as modified/coordinated ions. The fluoroaluminosilicate glass may be also a composition in which a lanthanoid series element is incorporated into the skeleton in order to impart further radiopacity. This lanthanoid series element also participates in the composition as a modified/coordinated ion.

As the inorganic filler, a hydrophobized inorganic fine particle may be contained. The average particle diameter

of the primary particles of the hydrophobic inorganic fine particle is preferably 0.1 to 50 nm, and the hydrophobization is preferably treated with a silane coupling agent and/or a modified silicone oil. By compounding, it can be expected to suppress the sedimentation of the inorganic filler and impart rheological characteristics in addition to improving the flexural strength.

Specific examples of the organic filler include polymers such as polymethyl methacrylate, polyethyl methacrylate, methyl methacrylate-ethyl methacrylate copolymer, ethyl methacrylate-butyl methacrylate copolymer, methyl methacrylate-trimethylolpropane methacrylate copolymer, polyvinylchloride, polystyrene, chlorinated polyethylene, nylon, polysulfone, polyethersulfone and polycarbonate.

Specific examples of the organic/inorganic composite filler include one obtained by covering the surface of a filler with a polymerizable monomer by polymerization, one obtained by mixing a filler and a polymerization monomer and polymerizing the monomer and thereafter grinding the resultant to a proper particle size, one obtained by dispersing a filler in a polymerizable monomer in advance for emulsion polymerization or suspension polymerization, one obtained by spray-drying a polymerizable monomer and a solvent which are dispersed with a filler in advance and polymerizing, and one obtained by spray-drying a solvent which is dispersed with a filler in advance, impregnating a polymerizable monomer and polymerizing, but are not limited thereto at all.

The feature of the ion sustained release glass is that at least one of fluorine ion, strontium ion, borate ion, aluminum ion and silicate ion is sustained release. Among these ions, it is preferable that a plurality of these ions are released at the same time.

As the ion sustained release glass used in the present invention, any ion sustained release glass can be used without any limitation as long as such ion sustained release glass includes one or more kinds of glass skeleton forming elements which form a glass skeleton, and one or more kinds of glass modifying elements which modify the glass skeleton. These ion sustained release glasses may be used alone or in combination of two or more thereof. Further, in the present disclosure, glass amphoteric elements that play a role of either a glass skeleton forming element or a glass modifying element depending on the glass composition are included within a category of a glass skeleton forming element. Specific examples of the glass skeleton forming element contained in the ion sustained release glass, include silica, aluminum, boron and phosphorus, and these can be used not only singly but also in combinations of a plurality thereof. Specific examples of the glass modifying element include halogen elements such as fluorine, bromine and iodine, alkali metal elements such as sodium and lithium, and alkali earth metal elements such as calcium and strontium, and these can be used not only singly but also in combinations of a plurality thereof. Among them, a glass composition including silica, aluminum or boron as the glass skeleton forming element and including fluorine, sodium or strontium as the glass modifying element is preferable, and specific examples include a silica glass, a fluoroaluminosilicate glass, a fluoroborosilicate glass and a fluoroalumino borosilicate glass containing strontium or sodium. Furthermore, from the viewpoint of sustainably releasing fluorine ion, strontium ion, borate ion or aluminum ion, a fluoroalumino borosilicate glass containing strontium is more preferable. Example of the glass composition range thereof is as follows: $SiO_2$: 15 to 35% by mass, $Al_2O_3$: 15 to 30% by mass, $B_2O_3$: 5 to 20% by mass, SrO: 20 to 45% by mass, F: 5 to 15% by mass, and $Na_2O$: 0 to 10% by mass. The glass composition can be confirmed by using an instrumental analysis such as an elementary analysis, Raman spectrum, and fluorescence X-ray analysis, and there is no problem as long as the actual measurement by any analysis methods matches to these composition ranges.

A method for preparing an ion sustained release glass is not particularly limited, and the glass can be produced by a preparing method such as a melting method or a sol-gel method. Among them, a preparing method by a melting method using a melting furnace is preferable from the viewpoint of ease of design of the glass composition, including raw material selection. The ion sustained release glass used in the present disclosure has an amorphous structure, but there is no problem even if it contains a partially crystalline structure, and further, a mixture of a glass having an amorphous structure and a glass having a crystal structure may be used without any problem. Whether the structure of the glass is an amorphous structure can be confirmed using an X-ray diffraction analysis or an analysis instrument such as a transmission electron microscope. In particular, the ion sustained release glass used in the present disclosure sustainably releases various ions by means of an equilibrium relation with ion concentrations in the external environment, and therefore an amorphous structure which is a homogeneous structure is preferable.

Further, in order to enhance ion sustained release property of the ion sustained release glass, it is preferable embodiment to functionalize the glass surface by a surface treatment, thereby improving the ion sustained-release property. Specific examples of a surface treatment material used in the surface treatment include a surfactant, a fatty acid, an organic acid, an inorganic acid, a monomer, a polymer, various coupling materials, a silane compound, a metal alkoxide compound, and a partially condensed product thereof. Among these surface treatment materials, it is preferable to perform composite surface treatment using an acidic polymer and a silane compound.

The composite surface treatment is a method for surface treatment by using an acidic polymer after coating the surface of the ion sustained release glass with a silane compound. Specific example is described in the following. A silane compound represented by the formula (5) is mixed in an aqueous dispersion containing an ion sustained release glass finely pulverized to a desired average particle diameter (D50) by pulverization or the like. The mixture is hydrolyzed

or partially hydrolyzed in the system to prepare a silanol compound. Then the silanol compound is condensed to form a polysiloxane, and then the surface of the ion sustained release glass is coated with the polysiloxane to obtain a polysiloxane coated ion sustained release glass.

[Formula (4)]

[Chemical formula 6]

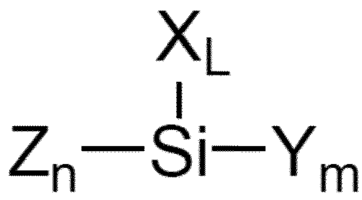

(in the formula, Z is $RO^-$, X is halogen, Y is $OH^-$, R is an organic group whose carbon number is less than or equal to 8, and n, m, and L are each an integer from 0 to 4 where $n + m + L = 4$)

Specific Examples of the silane compound represented by the formula (4) include tetramethoxysilane, tetraethoxysilane, tetrapropoxysilane, tetraallyloxysilane, tetrabutoxysilane, tetrakis (2-ethylhexyloxy) silane, trimethoxychlorosilane, triethoxychlorosilane, triisopropoxychlorosilane, trimethoxyhydroxysilane, diethoxydichlorosilane, tetraphenoxysilane, tetrachlorosilane and silicon hydroxide (silicon oxide hydrate), more preferable include tetramethoxysilane and tetraethoxysilane.

A low condensed product of the silane compound represented by the formula (4) is more preferable. Examples include a low condensed silane compound obtained by partial hydrolysis and condensation of tetramethoxysilane and tetraethoxysilane. These compounds can be used singly or in combination. An organosilane compound can also be added in the polysiloxane treatment as a part of the silane compound represented by the formula (4).

The polysiloxane coated ion sustained release glass, obtained in the above step, can be subjected to an acidic polymer treatment for a reaction with an acidic polymer to thereby provide an ion sustained release glass. The acidic polymer treatment can be conducted by using an equipment commonly used in the art as long as the equipment is a dry fluid type stirring machine, and examples include a Henschel mixer, a super mixer and a high-speed mixer. The reaction of the ion sustained release glass formed with the polysiloxane film and an acidic polymer can be conducted by contacting an acidic polymer solution by impregnating, spraying or the like. As an example, it is only necessary to perform a dry fluid of the polysiloxane coated ion sustained release glass, dispersing the acid polymer solution from above in the flow state, and sufficiently stirring. The method for dispersing the acidic polymer solution is not particularly limited, and a dropping or spraying method that can uniformly disperse is more preferable. The reaction is preferably conducted around room temperature, and when the temperature increases, there is a case where the reaction of an acid reactive element and the acidic polymer is fast, and thereby formation of an acidic polymer layer is not uniform.

It is preferable to remove the water content in the cement reaction phase by performing a heat treatment after the reaction. If water content remains in the cement reaction phase, it is disadvantageous in terms of strength, but since the filler in the present invention is covered and strengthened by the coupling agent condensate film, the decrease in mechanical strength is suppressed. The heat treatment method after the acid polymer treatment is not particularly limited, and can be performed by a known general method. As the equipment used for the heat treatment, a box-type hot air dryer or the like, a rotary heat treatment device capable of uniform heating and the like are preferable. The heat treatment temperature is in the range from room temperature to 200 °C, more preferably in the range from 40 to 150 °C. When the temperature is lower than this range, there is a risk that the removal of the aqueous medium is insufficient, and when the temperature is higher than this range, there is a risk that the organic layer of the acidic polymer is decomposed or discolored. Since the heat treatment period depends on the capacity of the dryer and the like, there is no problem as long as the aqueous medium can be sufficiently removed. After a heat treatment, a heat-treated product can be easily deagglomerated by application of a shear force or an impact force, and the deagglomerating method can be performed by, for example, the equipment used in the above reaction.

A solvent used in preparing the acid polymer solution used for the reaction may be solvent which dissolves the acid polymer without any problems. Examples of the solvent include water, methanol, ethanol, and acetone. Of these, water is particularly preferable. When water is used, an acid group of the acid polymer dissociates and reacts uniformly with the surface of the basic filler as the core.

The weight average molecular weight of the polymer dissolved in the acid polymer solution is in the range of 2000 to 50000, and preferably in the range of 5000 to 40000. In the case of treating with the acidic polymer having a weight average molecular weight of less than 2000, there is a tendency that an acidic polymer reaction phase is not

formed in the ion sustained release glass, and as a result, the ion sustained release property is low. On the other hand, in the case of treating with an acidic polymer having a weight average molecular weight of more than 50000, the viscosity of the acidic polymer solution becomes high, and therefore it may be difficult to uniformly treat the polysiloxane coated ion sustained release glass. The concentration of the acidic polymer in the acidic polymer solution is preferably in the range from 3 to 25 % by mass, more preferably in the range from 8 to 20 % by mass. When the concentration of the acidic polymer is less than 3 % by mass, the above described acidic polymer reaction phase is brittle and therefore the effect of improving the sustained release of ions cannot be obtained. When the concentration of the acidic polymer is more than 25 % by mass, the polysiloxane layer (porous) is difficult to diffuse in a uniform state, a homogeneous acidic polymer reaction phase is not obtained, and the reaction occurs immediately after contact with the polysiloxane coated ion sustained release glass, and therefore there is a problem that strongly reacted agglomerates generates. The addition amount of the acidic polymer solution to the polysiloxane coated ion sustained release glass is preferably in the range from 6 to 40 % by mass, more preferably 10 to 30 % by mass. Converting in this addition amount, an optimal amount of the acid polymer with respect to the polysiloxane coated ion sustained release glass is in the range of 1 to 7 % by mass, and an optimal amount of water is in the range of 10 to 25 % by mass.

As the acid polymer that can be used to form the acid polymer reaction phase on the surface of the polysiloxane coated ion sustained release glass by the method described above, any copolymers or homopolymers as long as the copolymer or homopolymer is a copolymer or homopolymer of a polymerizable monomer having an acid group such as a phosphoric acid residue, a pyrophosphoric acid residue, a thiophosphoric acid residue, a carboxylic acid residue, or a sulfonic acid residue. Specific examples of these polymerizable monomers include acrylic acid, methacrylic acid, 2-chloroacrylic acid, 3-chloroacrylic acid, aconitic acid, mesaconic acid, maleic acid, itaconic acid, fumaric acid, glutaconic acid, citraconic acid, 4-(meta) acryloyloxy ethoxycarbonyl phthalic acid, 4-(meta) acryloyloxy ethoxycarbonyl phthalic anhydride, 5-(meta) acryloylamino pentylcarboxylic acid, 11-(meth) acryloyloxy-1,1-undecane dicarboxylic acid, 2-(meta) acryloyloxyethyl dihydrogen phosphate, 10-(meta) acryloyloxydecyl dihydrogen phosphate, 20-(meta) acryloyloxyeicosil dihydrogen phosphate, 1,3-di (meth) acryloyloxypropyl-2-dihydrogen phosphate, 2-(meta) acryloyloxyethyl phenyl phosphate, 2-(meta) acryloyloxyethyl-2'-bromoethyl phosphate, (meta) acryloyloxyethyl phenylphosphonate, [2-(meth) acryloyloxyethyl] pyrophosphate, 2-(meta) acryloyloxyethyl dihydrogen dithiophosphosphate and 10-(meta) acryloyloxydecyl dihydrogen thiophosphate. Of these polymers which are (co)polymer of these polymerizable monomers, a homopolymer or a copolymer of $\alpha$-$\beta$ unsaturated carboxylic acid that is relatively slow in acid-base reaction with an acid reactive element contained in the polysiloxane coated ion sustained release glass is preferable, and specific examples include an acrylic acid polymer, an acrylic acid-maleic acid copolymer, and an acrylic acid-itaconic acid copolymer.

The above described (E) filler can be treated with a surface treatment material represented by a silane coupling material in order to improve the affinity to the polymerizable monomer, the dispersability in the polymerizable monomer, and the mechanical strength and water resistance of the cured body. The surface treatment material and the surface treatment method are not particularly limited, and known methods such as a method of spraying the surface treatment material while stirring the powdery filler, a method of dispersing and mixing the filler and the surface treatment material in a solvent, and a method of supplying a vapor or gaseous silane coupling material to the filler surface, can be adopted without limitation. As a silane coupling material used for surface treatment of the filler, methyltrimethoxysilane, methyltriethoxysilane, methyltrichlorosilane, dimethyldichlorosilane, trimethylchlorosilane, vinyltrichlorosilane, vinyltriethoxysilane, vinyltris (2-methoxyethoxy) silane, 3-methacryloyloxypropyl trimethoxysilane, 3-chloropropyl trimethoxysilane, 3-glycidoxypropyl trimethoxysilane, 3-(meth) acryloxypropyl trimethoxysilane, 8-(meth) acryloxyoctyl trimethoxysilane, 11-(meth) acryloxiundecyl trimethoxysilane, hexamethyldisilazane and the like are preferable. In addition to the silane coupling material, surface treatment of the filler can be performed by a method using a titanate coupling material or an aluminate coupling material. The treatment amount of the surface treatment material in the filler is preferably 0.01 to 30 parts by mass, more preferably 0.5 to 20 parts by mass with respect to 100 parts by mass of the filler before treatment.

The shape of the (E) filler is not particularly limited, and any shape of the filler such as a spherical shape, a needle shape, a plate shape, a crushed shape or a scale shape can be used. The average particle diameter of the filler is preferably 0.01 $\mu$m to 50 $\mu$m, more preferably 0.01 $\mu$m to 30 $\mu$m, still more preferably 0.05 $\mu$m to 20 $\mu$m, and more preferably 0.05 $\mu$m to 10 $\mu$m.

The shape of the (E) filler is not particularly limited, and any shape of the filler such as a spherical shape, a needle shape, a plate shape, a crushed shape or a scale shape can be used. The average particle diameter of the filler is preferably 0.01 $\mu$m to 50 $\mu$m, more preferably 0.01 $\mu$m to 30 $\mu$m, still more preferably 0.05 $\mu$m to 20 $\mu$m, and more preferably 0.05 $\mu$m to 10 $\mu$m.

The dental curable composition of the present invention preferably contains a hydrophobic silica fine particle having an average particle diameter of the primary particles of 1 to 40 nm as the (E) filler in order to impart rheological property. The primary particle diameter indicates the diameter of one particle (primary particle) constituting the powder. In the present invention, the average particle diameter may be, for example, an average particle diameter calculated based on a volume-based grain size distribution measured by a laser diffraction type particle size distribution measuring device or the like, and may be measured by, for example, a laser diffraction type grain size measuring apparatus

(Microtrac MT3300EXII: NIKKISO Co., Ltd.). In addition, the primary particle diameter can be measured by dynamic light scattering particle size measurement and can be measured by using electron micrographs for the case that primary particles are strongly aggregated to form secondary particles. It is preferably that the average particle diameter of the primary particles of the silica fine particle is 1 to 40 nm. Examples of a method for hydrophobizing a silica fine particle include surface treatment with a modified silicone oil such as dimethylsilicone oil and/or surface treatment with a silane coupling material which has an alkylsilyl group and may have a trimethylsilyl group, a dimethylsilyl group, a methylsilyl group or a (meth) acryloyl group having an alkyl chain having 3 or more and 18 or less carbon atoms.

Specific examples of the hydrophobic silica fine particle include Aerosil R972, Aerosil R974, Aerosil R976, Aerosil R711, Aerosil R7200, Aerosil R976S, Aerosil R202, Aerosil R812, Aerosil R812S, Aerosil R805, Aerosil R8200, Aerosil R104, Aerosil R106, Aerosil RY200, Aerosil RX200, Aerosil RY200S, Aerosil RA200H and Aerosil RA200HS, which are manufactured by Nippon Aerosil Co., Ltd. and commercially available under the trade name of Aerosil.

By containing the hydrophobic silica fine particle having the average particle diameter of the primary particles of 1 to 40 nm, the dental curable composition exhibits appropriate thixotropy and thus has good operability. In the dental curable composition of the present invention, since the dental curable composition itself exhibits good storage stability, good storage stability can be expected even if the compounding amount of the filler in the dental curable composition is small. Generally, when the compounding amount of the filler is large, there is a case where the transparency is lowered and the elasticity and the flexibility is lowered. There is a case where transparency, elasticity and flexibility are required in a dental coating material, a dental lining material, a dental cement, a dental bonding material, a dental splinting material and a dental manicure material. When the compounding amount of the filler in the dental curable composition is reduced in order to avoid such a case, there is a case where the operability is deteriorated. In such a case, the hydrophobic silica fine particle having an average particle diameter of the primary particles of 1 to 40 nm is preferably used because it is expected to impart rheological property and to improve operability by small compounding amount. It is preferable that the dental curable composition of the present invention contains 1 to 30 parts by mass of the hydrophobic silica fine particle having the average particle diameter of the primary particles of 1 to 40 nm with respect to 100 parts by mass of the (A) polymerizable monomer. When the compounding amount is less than 1 part by mass, there is a case where the rheological property is not exhibited. When the compounding amount is more than 30 parts by mass, there is a case where the operability of the dental curable composition is deteriorated.

In the present invention, a paste-like dental curable composition may be prepred by preparing a matrix, which is a medium having a lower viscosity than a paste and contains a polymerizable monomer, a photopolymerization initiator, a fluorescent agent, an ultraviolet absorber, and, if necessary, a portion of a filler, and then mixing the prepared matrix and filler. By preparing the paste in this manner, it it possible to dissolve a photopolymerization initiator, a fluorescent agent, an ultraviolet absorbing agent and the like in a polymerizable monomer, and to uniformly disperse the photopolymerization initiator in a paste. In particular, solids at room temperature may cause sedimentation and performance variation in the case of not be dissolved in the matrix. In the case of dispersing uniformly without sedimentation in the matrix, a filler having the primary particles diameter exceeding 100 nm may be compounded after compounding a filler having the primary particles diameter of 100 nm or less. Since it is preferable to contain a process for confirming that the matrix has been dissolved in manufacturing, it is preferable that the matrix does not contain a filler that does not dissolve in the matrix.

The dental curable composition of the present invention may contain 0 to 900 parts by mass of the (E) filler with respect to 100 parts by mass of the (A) polymerizable monomer. In the case of a dental composite resin for filling as one embodiment of the present invention, the compounding amount of the filler (E) is preferably 100 to 900 parts by mass. When the compounding amount of the filler (E) is less than 100 parts by mass, there is a case where the mechanical property is deteriorated or the operability is poor. When the compounding amount of the filler (E)is more than 900 parts by mass, there is a case where the operability of the dental curable composition is deteriorated.

<Other component>

Further, the dental curable composition of the present invention may contain a component other than above described (A) to (D) components within a range not to impair the effect of the present invention. For example, an excipient typified by fumed silica, a polymerization inhibitor such as hydroquinone, hydroquinone monomethyl ether and 2,5-ditershally butyl-4-methylphenol, a chain transfer agent such as $\alpha$-alkylstyrene compound, mercaptan compound such as n-butyl mercaptan and n-octyl mercaptan, and terpenoid compound such as limonene, myrsen, $\alpha$-terpinene, $\beta$-terpinene, $\gamma$-terpinene, terpinoren, $\beta$-pinene and $\alpha$-pinene, a metal supplementary agent such as an aminocarboxylic acid chelating agent and a phosphonic acid chelating agent, a discoloration inhibitor, an antibacterial agent, coloring pigment, water and solvent that can be mixed with water in any ratio, and other additives conventionally known in the art may be added as necessary and as desired.

The dental curable composition of the present invention may comprise only the (A) polymerizable monomer, the (B) polymerization initiator, the (C) fluorescent agent and the (D) ultraviolet absorber. Further, as the components

other than the (A) to (D), only one or more of the above-mentioned components may be contained.

A preparing method of the dental curable composition of the present invention is not particularly limited. Examples of a general preparing method of a dental curable composition include a method which comprises preparing a matrix by the (A) polymerizable monomer, the (B) polymerization initiator, the (C) fluorescent agent and the (D) ultraviolet absorber by a known method such as a planetary centrifugal mixer, a tumbler mixer, a mix rotor, a dissolver and a planetary mixer in advance, kneading the matrix and the (E) filler by a known method such as a planetary centrifugal mixer, a tumbler mixer, a mix rotor, a dissolver and a planetary mixer, and removing air bubbles under reduced pressure to prepare a uniform paste. In the present disclosure, it can be prepared by the above-described method without any problem.

It is preferable that the dental curable composition of the present invention is used for a dental adhesive material, a dental composite resin, a dental core build-up material, a dental resin cement, a dental coating material, a dental sealant material, a dental manicure material, a dental splinting material, a dental cutting and machining material and a dental 3D printer material, and it is particularly preferable to use for a dental adhesive material, a dental composite resin, a dental core build-up material, a dental resin cement, a dental coating material, a dental sealant material, a dental manicure material, a dental splinting material and a dental orthodontic material.

[Example]

Hereinafter, example of the present invention are specifically described. However, the present invention is not intended to be limited to these Examples.

The materials used in Examples and Comparative examples and their abbreviations are listed below.

[(A) Polymerizable monomer]

<Radical polymerizable monomer>

Bis-GMA: 2,2-bis [4-(3-methacryloyloxy-2-hydroxypropoxy) phenyl] propane
2.6E: 2,2-bis (4-(meth) acryloyloxy polyethoxyphenyl) propane in which the average addition mole number of ethoxy groups is 2.6
UDMA: N,N-(2,2,4-trimethyl hexamethylene) bis [2-(aminocarboxy) ethanol] methacrylate NPG: neopentyl glycol dimethacrylate
TEGDMA: triethyleneglycol dimethacrylate
HEMA: hydroxyethyl methacrylate

<(A1) polymerizable monomer having an acidic group>

MDP: 10-methacryloyloxydecyl dihydrogen phosphate
MHPA: 6-methacryloxyhexyl phosphonoacetate

[(B) Polymerization initiator]

[(B-1) Photosensitizer]

CQ: camphorquinone
BAPO: phenyl bis (2,4,6-trimethylbenzoyl) phosphine oxide

[(B-2) Photoacid generator]

DPI: diphenyl iodonium hexafluorophosphate

[Chemical formula 7]

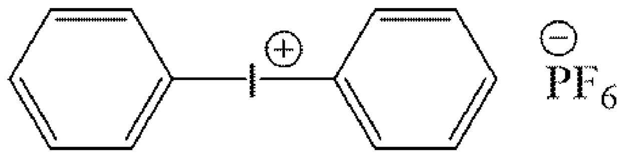

IFP: p-cumenyl (p-tolyl) iodonium tris (pentafluoroethyl) trifluorophosphate

[Chemical formula 8]

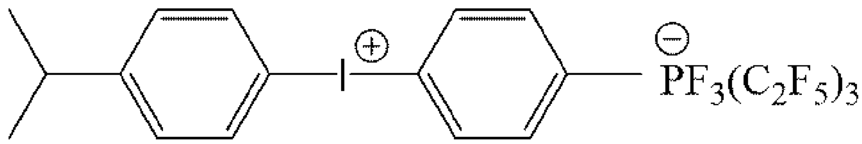

IFB: bis (4-tert-butylphenyl) iodonium tetrakis pentafluoro phenylborate

[Chemical formula 9]

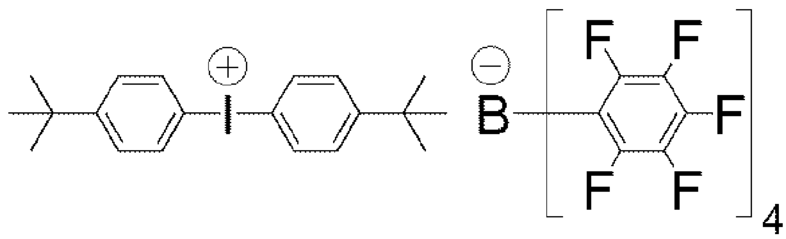

IFG: bis (4-tert-butylphenyl) iodonium tetrakis pentafluoro phenylgallate

[Chemical formula 10]

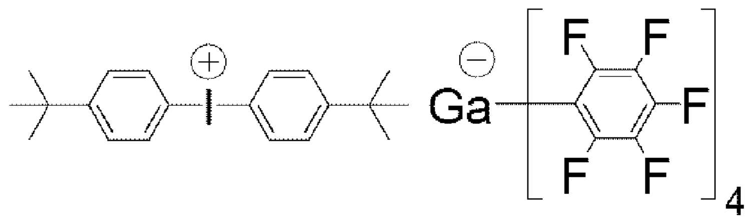

[(B-3) Polymerization accelerator]

<Aliphatic tertiary amine>

TBA: tribenzylamine
DM: 2-(N,N-dimethylamino) ethyl methacrylate

<Aromatic tertiary amine>

DMBE: N,N-dimethylaminobenzoic acid ethyl

[(C) Fluorescent agent]

DHT: diethyl-2,5-dihydroxy terephthalate
TB: 2,5-thiophenediyl bis(5-tert-butyl-1,3-benzoxazole)

[(D) Ultraviolet absorber]

Samples were prepared by dissolving various UV absorbers in acetonitrile to a concentration of 100 ppm, and the absorbance of each sample was measured in the wavelength range of 250 nm to 400 nm using a ultraviolet-visible spectrophotometer (V-750, manufactured by JASCO Corporation). Each ultraviolet absorbent was classified based on the absorbance measurement results.

«Ultraviolet absorber having an absorption maximum only in the range of 250 nm or more and less than 320 nm»

D1: benzophenone
D2: 4-tert-butylphenyl salicylate

«Ultraviolet absorber having an absorption maximum only in the range of 320 nm or more and less than 400 nm»

D3: 1,4-naphthoquinone

«Ultraviolet absorber having the ratio of an absorbance of the first absorption maximum to an absorbance of the second absorption maximum of less than 1.0»

D4: 2,2'-dihydroxy-4,4'-dimethoxybenzophenone (the ratio of an absorbance of the first absorption maximum to an absorbance of the second absorption maximum: 0.73)
D5: 2-[2-hydroxy-5-[2-(methacryloyloxy)-ethyl]phenyl]-2H-benzotriazole (the ratio of an absorbance of the first absorption maximum to an absorbance of the second absorption maximum: 0.79)
D6: 2-(2H-benzotriazol-2-yl)-p-cresol (the ratio of an absorbance of the first absorption maximum to an absorbance of the second absorption maximum: 0.84)

«Ultraviolet absorber having the ratio of an absorbance of the first absorption maximum to an absorbance of the second absorption maximum within the range of 1 to 4»

D7: 2-hydroxy-4-n-octyloxybenzophenone (the ratio of an absorbance of the first absorption maximum to an absorbance of the second absorption maximum: 1.47)
D8: 2-(4,6-diphenyl-1,3,5-triazin-2-yl)-5-[2-(2-ethylhexanoyloxy) ethoxy] phenol (the ratio of an absorbance of the first absorption maximum to an absorbance of the second absorption maximum: 2.09)

[(E) Filler]

The preparing method of each filler used for preparing the dental curable composition is shown below.

(Filler E1)

A silane coupling treatment solution prepared by stirring 50.0 g of water, 35.0 g of ethanol, and 3.0 g of 3-methacryloyloxypropyl trimethoxysilane as a silane coupling material at room temperature for 2 hours was added to 100.0 g of fluoroalumino borosilicate glass (average particle diameter: 1.0 μm) and stirred for 30 minutes. Thereafter, a heat treatment was performed at 100 °C for 15 hours to obtain a filler E1.
A silane coupling treatment solution prepared by stirring 50.0 g of water, 35.0 g of ethanol, and 5.0 g of 3-methacryloyloxypropyl trimethoxysilane as a silane coupling material at room temperature for 2 hours was added to 100.0 g of the zirconium silicate filler (average particle diameter: 0.8 μm, zirconia: 85 wt.%, silica: 15 wt.%) and stirred for 30 minutes. Thereafter, a heat treatment was performed at 100 °C for 15 hours to obtain filler E2.

(Filler E3)

After various raw materials of silica dioxide, aluminum oxide, boron oxide, sodium fluoride, and strontium carbonate were mixed, the mixture was melted to obtain glass A (glass composition: SiO2 22.5% by mass, Al2O3 20.0% by mass, B2O3 12.3% by mass, SrO 35.7% by mass, Na2O 2.5% by mass, and F 7.0% by mass). The obtained glass A was pulverized using a vibration mill for 100 hours, and then pulverized using a wet bead mill for 3 hours. Then, 4.5 g of a low condensate of silane compound "MKC SILICATE MS56S" (SiO2 content: 56.0% by mass, degree of polymerization: 2 to 100, manufactured by Mitsubishi Chemical Corporation) was added to 100 g of the obtained pulverized product, and stirred-mixed for 90 minutes. After mixing for a predetermined time, the resulting treated slurry was aged in a hot air dryer at 50 °C for 40 hours, then heated to 150 °C and held for 6 hours, then cooled to obtain a heat treated product. The obtained heat-treated product was placed in a Henschel mixer and pulverized at 1800 rpm for 5 minutes. After pulverization, a polysiloxane-treated material having good fluidity was obtained.

(Acidic polymer treatment)

Then, 100 g of the polysiloxane-treated product was put into a Henschel mixer, and 16.0 g of an aqueous polyacrylic acid solution (polymer concentration: 13% by mass, weight average molecular weight: 20,000; manufactured by Nacalai Co., Ltd.) was sprayed from above while stirring. After spraying, the powder taken out from the mixer was heated in a hot air dryer at 100 °C for 3 hours to obtain a polysiloxane-polyacrylic acid-treated product.

(Silane treatment)

A silane coupling treatment solution prepared by stirring 100.0 g of water, 80.0 g of ethanol, 0.003 g of phosphoric acid, and 12.0 g of 8-methacryloyloxypropyl trimethoxysilane as a silane coupling agent at room temperature for 2 hours was added to 100.0 g of the polysiloxane-polyacrylic acid-treated product and stirred for 30 minutes. Thereafter, a heat treatment was performed at 100 °C for 15 hours to obtain filler E3.

A silane coupling treatment solution prepared by stirring 100.0 g of water, 80.0 g of ethanol, 0.003 g of phosphoric acid, and 12.0 g of 8-methacryloyloxy octyltrimethoxysilane as a silane coupling agent at room temperature for 2 hours was added to 100.0 g of the above polysiloxane-treated product and stirred for 30 minutes. Thereafter, a heat treatment was performed at 100 °C for 15 hours to obtain filler E3.

(Filler E4)

A uniform matrix was prepared by mixing 60 parts by mass of BisGMA, 40 parts by mass of TEGDMA and 0.2 parts by mass of BPO. The prepared matrix, 300 parts by mass of fluoroboroaluminosilicate glass (average primary particle diameter of 0.5 μm), 20 parts by mass of Aerosil R7200 and 15 parts by mass of γ-methacryloxypropyl trimethoxysilane were mixed and polymerized in a nitrogen atmosphere using a kneader to prepare a cured body. The cured body was coarsely crushed using a roll crusher and then finely pulverized using a vibration mill to prepare a finely pulverized product having an average particle diamter of 20 μm.

A mixture was prepared by mixing 100 parts by mass of the sieved finely pulverized product, 1 part by mass of water, 1 part by mass of ethanol, and 6 parts by mass of γ-methacryloxypropyltrimethoxysilane, and the mixture was dried at 90 °C for 10 hours to prepare an organic-inorganic composite filler.

(Filler E5)

Aerosil R8200 (manufactured by Evonik)

(Filler E6)

Aerosil R711 (manufactured by Evonik)

(Filler E7)

Aerosil AluC805 (manufactured by Evonik)

[Polymerization inhibitor]

MeHQ: p-methoxyphenol
BHT: butylated hydroxytoluene

[Chemical polymerization initiator]

BPO: benzoyl peroxide
TMBH: 1,1,3,3-tetramethylbutyl hydroperoxide
PTU: pyridyl thiourea
DEPT: p-tolyldiethanolamine
VOA: vanadyl acetylacetonate
CAA: acetylacetone copper
KPS: potassium peroxodisulfate

<Preparing method of one matrix>

All components shown in Tables 1 to 2 other than the (E) filler were put into a wide mouthed plastic container and mixed by using a mix rotor VMRC-5 under the condition of 50 °C and 100 rpm for 48 hours to prepare a matrix. In the tables 1 to 2, the content (parts by mass) of each component is indicated by the numerical value in parentheses after the abbreviation of each component.

<Preparing method of one pack type dental curable composition>

All components shown in Tables 1 to 2 other than the (E) filler were mixed to prepare a matrix. Then, the matrix and the (E) filler were kneaded in a planetary mixer, and filled into a syringe made of PP to prepare a dental curable composition. In the tables 1 to 2, the content (parts by mass) of each component is indicated by the numerical value in parentheses after the abbreviation of each component.

<Preparing method of two packs type dental curable composition>

All components shown in Table 3 other than the (E) filler were mixed to prepare a matrix. Then, the matrix and the (E) filler were kneaded in a planetary mixer to prepare a first paste and a second paste separately, and filled into a double syringe (5 mL) manufactured by Mixpack Co., Ltd. to prepare a dental curable composition. In the table 3, the content (parts by mass) of each component is indicated by the numerical value in parentheses after the abbreviation of each component. Further, Examples of two packs type dental curable composition were evaluated after kneading by SHOFU mixing tip manufactured by SHOFU INC.

[Table 1]

| Examples | Matrix | | | | | | | | | (E) filler |
|---|---|---|---|---|---|---|---|---|---|---|
| | (A) Polymerizable monomer | | (B) Polymerization initiator | | | Polymerization inhibitor | (C) Fluorescent agent | (D) Ultraviolet absorber | | |
| | Polymerizable monomer other than (A1) | (A1) Polymerizable monomer having an acidic group | (B-1) Photosensitizer | (B-2) Photoacid generator | (B-3) Polymerization accelerator | | | | | |
| Example 1 | Bis-GMA(60), TEGDMA(40) | - | CQ(0.1) | IFB(3) | TBA(3) | MeHQ(0.01) | DHT(0.01) | D7 (0.005) | | E1(230),E3 (100), E5 (5),E7(6) |
| Example 2 | Bis-GMA(60), TEGDMA(40) | - | CQ(0.1) | IFB(3) | TBA(3) | MeHQ(0.01) | DHT(0.01) | D7 (0.01) | | E1(230),E3 (100), E5 (5),E7(6) |
| Example 3 | Bis-GMA(60), TEGDMA(40) | - | CQ(0.1) | IFP(3) | TBA(3) | MeHQ(0.01) | DHT(0.01) | D7 (0.05) | | E1(230),E3 (100), E5 (5),E7(6) |
| Example 4 | Bis-GMA(60), TEGDMA(40) | - | CQ(0.1) | IFB(3) | DM(3) | MeHQ(0.01) | DHT(0.01) | D7 (0.1) | | E1(230),E3 (100), E5 (5),E7(6) |
| Example 5 | Bis-GMA(60), TEGDMA(40) | - | CQ(0.1) | IFP(3) | TBA(3) | MeHQ(0.01) | DHT(0.01) | D7 (0.1) | | E1(230),E3 (100), E5 (5),E7(6) |
| Example 6 | Bis-GMA(60), TEGDMA(40) | - | CQ(0.1) | IFG(3) | TBA(3) | MeHQ(0.01) | DHT(0.01) | D7 (0.1) | | E1(230),E3 (100), E5 (5),E7(6) |
| Example 7 | Bis-GMA(60), TEGDMA(40) | - | CQ(0.1) | DPI(0.5) | DM(3) | MeHQ(0.01) | DHT(0.01) | D7 (0.1) | | E1(230),E3 (100), E5 (5),E7(6) |
| Example 8 | Bis-GMA(45), UDMA(30), NPG (25) | - | CQ(0.1) | IFB(0.2) | TBA(0.2) | MeHQ(0.01) | DHT(0.01) | D7(0.5) | | E1(320),E3 (80), E6(3) |
| Example 9 | Bis-GMA(45), UDMA(30), NPG (25) | - | CQ(0.1) | IFP(0.1) | TBA(0.2) | MeHQ(0.01) | DHT(0.01) | D7(0.5) | | E1(320),E3 (80), E6(3) |

(continued)

| Examples | Matrix | | | | | | | | (E) filler |
|---|---|---|---|---|---|---|---|---|---|
| | (A) Polymerizable monomer | | (B) Polymerization initiator | | | Polymerization inhibitor | (C) Fluorescent agent | (D) Ultraviolet absorber | |
| | Polymerizable monomer other than (A1) | (A1) Polymerizable monomer having an acidic group | (B-1) Photosensitizer | (B-2) Photoacid generator | (B-3) Polymerization accelerator | | | | |
| Example 10 | Bis-GMA(45), UDMA(30), NPG (25) | - | CQ(0.1) | IFG(0.1) | TBA(0.2) | - | DHT(0.01) | D7(0.5) | E1(320),E3 (80), E6(3) |
| Example 11 | Bis-GMA(45), UDMA(30), NPG (25) | - | CQ(0.1) | DPI(0.2) | TBA(0.2) | - | DHT(0.01) | D7(0.5) | E1(320),E3 (80), E6(3) |
| Example 12 | Bis-GMA(45), UDMA(30), NPG (25) | - | CQ(0.1) | IFB(1) | TBA(3) | MeHQ(0.01) | DHT(0.01) | D7(0.5) | E1(320),E3 (80), E6(3) |
| Example 13 | Bis-GMA(45), UDMA(30), NPG (25) | - | CQ(0.1) | IFB(2) | TBA(6) | MeHQ(0.01) | DHT(0.01) | D7(0.5) | E1(320),E3 (80), E6(3) |
| Example 14 | Bis-GMA(45), UDMA(30), NPG (25) | - | CQ(0.4) | - | TBA(6) | MeHQ(0.01) | DHT(0.01) | D7(0.5) | E1(320),E3 (80), E6(3) |
| Example 15 | Bis-GMA(45), UDMA(30), NPG (25) | - | CQ(0.4) | - | DMBE(2) | - | DHT(0.01) | D7(0.5) | E1(320),E3 (80), E6(3) |
| Example 16 | Bis-GMA(45), UDMA(30), NPG (25) | - | CQ(0.1) | IFB(5) | TBA(8) | MeHQ(0.01) | DHT(0.01) | D7(0.5) | E1(320),E3 (80), E6(3) |
| Example 17 | Bis-GMA(45), UDMA(30), NPG (25) | - | CQ(0.1) | IFP(5) | TBA(8) | MeHQ(0.01) | DHT(0.01) | D7(0.5) | E1(320),E3 (80), E6(3) |
| Example 18 | Bis-GMA(45), UDMA(30), NPG (25) | - | CQ(0.1) | IFG(3) | TBA(8) | MeHQ(0.01) | DHT(0.01) | D7(0.5) | E1(320),E3 (80), E6(3) |

(continued)

| Examples | Matrix | | | | | | | | | (E) filler |
|---|---|---|---|---|---|---|---|---|---|---|
| | (A) Polymerizable monomer | | (B) Polymerization initiator | | | Polymerization inhibitor | (C) Fluorescent agent | (D) Ultraviolet absorber | | |
| | Polymerizable monomer other than (A1) | (A1) Polymerizable monomer having an acidic group | (B-1) Photosensitizer | (B-2) Photoacid generator | (B-3) Polymerization accelerator | | | | | |
| Example 19 | Bis-GMA(45), UDMA(30), NPG (25) | - | CQ(0.5) | DPI(0.5) | TBA(9) | MeHQ(0.01) | DHT(0.01) | D7(0.5) | | E1(320),E3 (80), E6(3) |
| Example 20 | Bis-GMA(45), UDMA(30), NPG (25) | - | CQ(0.2) | - | TBA(3) | - | DHT(0.01) | D7 (0.5) | | E1(320),E3 (80), E6(3) |
| Example 21 | Bis-GMA(45), UDMA(30), NPG (25) | - | CQ(0.2) | IFG(3) | TBA(3) | - | DHT(0.01) | D7(0.5) | | E1(320),E3 (80), E6(3) |
| Example 22 | Bis-GMA(45), UDMA(30), NPG (25) | - | CQ(0.2) | IFB(3) | DMBE(3) | - | DHT(0.01) | D7(0.5) | | E1(320),E3 (80), E6(3) |
| Example 23 | Bis-GMA(45), UDMA(30), NPG (25) | - | CQ(0.2) | - | DMBE(3) | - | DHT(0.01) | D7(0.5) | | E1(320),E3 (80), E6(3) |
| Example 24 | Bis-GMA(60), TEGDMA(40) | - | CQ(0.15) | IFB(2) | TBA(2) | MeHQ(0.01) | DHT(0.001) | D7(0.1) | | E1(230),E3 (100), E5 (5),E7(6) |
| Example 25 | Bis-GMA(60), TEGDMA(40) | - | CQ(0.1) | IFB(3) | DM(3) | MeHQ(0.01) | DHT(0.005) | D7(0.3) | | E1(230),E3 (100), E5 (5),E7(6) |
| Example 26 | Bis-GMA(60), TEGDMA(40) | - | CQ(0.1) | IFP(3) | TBA(3) | MeHQ(0.01) | DHT(0.05) | D7(0.5) | | E1(230),E3 (100), E5 (5),E7(6) |
| Example 27 | Bis-GMA(60), TEGDMA(40) | - | CQ(0.1) | IFB(4) | TBA(4) | MeHQ(0.01) | DHT(0.15) | D7(0.8) | | E1(230),E3 (100), E5 (5),E7(6) |

(continued)

| Examples | Matrix | | | | | | | | (E) filler |
|---|---|---|---|---|---|---|---|---|---|
| | (A) Polymerizable monomer | | (B) Polymerization initiator | | | Polymerization inhibitor | (C) Fluorescent agent | (D) Ultraviolet absorber | |
| | Polymerizable monomer other than (A1) | (A1) Polymerizable monomer having an acidic group | (B-1) Photosensitizer | (B-2) Photoacid generator | (B-3) Polymerization accelerator | | | | |
| Example 28 | UDMA(60), TEGDMA(30) | MHPA(10) | CQ(0.3) | IFB(2) | TBA(3) | - | DHT(0.01) | D7 (0.8) | E2(180),E3 (50), E5(3), E7(5) |
| Example 29 | UDMA(60), TEGDMA(30) | MHPA(10) | CQ(0.3) | IFP(3) | TBA(2) | - | DHT(0.01) | D7 (0.8) | E2(180),E3 (50), E5(3), E7(5) |
| Example 30 | UDMA(60), TEGDMA(30) | MHPA(10) | CQ(0.1) | IFB(4) | TBA(4) | MeHQ(0.01) | DHT(0.01) | D7 (1.0) | E2(180),E3 (50), E5(3), E7(5) |
| Example 31 | UDMA(60), TEGDMA(30) | MHPA(10) | CQ(0.1) | IFG(3) | TBA(3) | MeHQ(0.01) | DHT(0.01) | D7 (1.5) | E2(180),E3 (50), E5(3), E7(5) |
| Example 32 | UDMA(60), TEGDMA(30) | MHPA(10) | CQ(0.1) | IFP(2) | TBA(3) | MeHQ(0.01) | DHT(0.01) | D7 (2.0) | E2(180),E3 (50), E5(3), E7(5) |
| Example 33 | UDMA(60), TEGDMA(30) | MHPA(10) | CQ(0.1) | IFB(1.5) | TBA(1.5) | MeHQ(0.01) | TB(0.001) | D7(0.05) | E2(180),E3 (50), E5(3), E7(5) |
| Example 34 | UDMA(60), TEGDMA(30) | MHPA(10) | CQ(0.1) | IFB(3) | TBA(3) | MeHQ(0.01) | TB(0.004) | D7(0.15) | E2(180),E3 (50), E5(3), E7(5) |
| Example 35 | UDMA(60), TEGDMA(30) | MHPA(10) | CQ(0.1) | IFB(4) | TBA(4) | MeHQ(0.01) | TB(0.01) | D7(0.5) | E2(180),E3 (50), E5(3), E7(5) |

[Table 2]

| Examples | Matrix | | | | | | | | | (E) filler |
|---|---|---|---|---|---|---|---|---|---|---|
| | (A) Polymerizable monomer | | (B) Polymerization initiator | | | Polymerization inhibitor | (C) Fluorescent agent | (D) Ultraviolet absorber | | |
| | Polymerizable monomer other than (A1) | (A1) Polymerizable monomer having an acidic group | (B-1) Photosensitizer | (B-2) Photoacid generator | (B-3) Polymerization accelerator | | | | | |
| Example 36 | UDMA(60), TEGDMA(30) | MHPA(10) | CQ(0.1) | IFP(4) | TBA(3) | MeHQ(0.01) | TB(0.05) | D7(0.5) | | E2(180),E3 (50), E5(3), E7(5) |
| Example 37 | UDMA(60), TEGDMA(30) | MHPA(10) | CQ(0.1) | IFB(3) | TBA(1.5) | MeHQ(0.01) | TB(0.1) | D7(0.8) | | E2(180),E3 (50), E5(3), E7(5) |
| Example 38 | UDMA(60), TEGDMA(30) | MHPA(10) | CQ(0.1) | IFG(2.5) | TBA(3) | MeHQ(0.01) | DHT(0.01) | D8(0.005) | | E2(180),E3 (50), E5(3), E7(5) |
| Example 39 | UDMA(60), TEGDMA(30) | MHPA(10) | CQ(0.1) | IFB(3) | TBA(2.5) | MeHQ(0.01) | DHT(0.01) | D8 (0.05) | | E2(180),E3 (50), E5(3), E7(5) |
| Example 40 | Bis-GMA(60), TEGDMA(40) | - | CQ(0.1) | IFB(3) | TBA(3) | MeHQ(0.01) | DHT(0.05) | D8 (0.5) | | E1(300),E3 (100), E4 (470),E6 (4),E7(3) |
| Example 41 | 2.6E(70), TEGDMA(30) | - | CQ(0.3) BAPO (0.3) | - | TBA(3) | - | DHT(0.05) | D8 (0.5) | | E1(230),E3 (100), E5 (5),E7(6) |
| Example 42 | Bis-GMA(60), TEGDMA(40) | - | CQ(0.1) | IFB(3) | TBA(8) | MeHQ(0.01) | DHT(0.05) | D8 (0.5) | | E1(300),E3 (100), E4 (470),E6 (4),E7(3) |
| Example 43 | UDMA(70), TEGDMA(30) | - | CQ(0.1) | IFB(3) | TBA(3) | MeHQ(0.01) | DHT(0.01) | D8(0.8) | | E2(150),E5 (8) |
| Example 44 | UDMA(70), TEGDMA(30) | - | CQ(0.3) | - | TBA(3) | - | DHT(0.01) | D8(0.8) | | E2(150),E5 (8) |

(continued)

| Examples | Matrix | | | | | | | | | (E) filler |
|---|---|---|---|---|---|---|---|---|---|---|
| | (A) Polymerizable monomer | | (B) Polymerization initiator | | | Polymerization inhibitor | (C) Fluorescent agent | (D) Ultraviolet absorber | | |
| | Polymerizable monomer other than (A1) | (A1) Polymerizable monomer having an acidic group | (B-1) Photosensitizer | (B-2) Photoacid generator | (B-3) Polymerization accelerator | | | | | |
| Example 45 | UDMA(70), TEGDMA(30) | - | CQ(0.3) | - | DMBE(3) | - | DHT(0.01) | D8(0.8) | | E2(150),E5 (8) |
| Example 46 | UDMA(50), NPG(35) | MDP(15) | CQ(0.1) | DPI(0.7) | TBA(3) | MeHQ(0.01) | DHT(0.01) | D8 (1.0) | | E2(180),E3 (50), E5(5) |
| Example 47 | 2.6E(70), TEGDMA(30) | - | CQ(0.1) | IFB(3) | TBA(3) | MeHQ(0.01) | DHT(0.01) | D8 (2.0) | | E1(230),E3 (100), E5 (5),E7(6) |
| Example 48 | UDMA(50), NPG(35) | MDP(15) | CQ(0.1) | IFB(3) | DM(3) | MeHQ(0.01) | DHT(0.001) | D8(0.05) | | E2(180),E3 (50), E5(5) |
| Example 49 | UDMA(70), TEGDMA(30) | - | CQ(0.1) | IFB(3) | TBA(3) | MeHQ(0.01) | DHT(0.05) | D8(0.5) | | E2(280),E5 (8) |
| Example 50 | UDMA(70), TEGDMA(30) | - | CQ(0.1) | DPI(0.5) | DM(5) | MeHQ(0.01) | DHT(0.1) | D8(0.7) | | E2(280),E5 (8) |
| Example 51 | Bis-GMA(60), TEGDMA(40) | - | CQ(0.1) | IFB(3) | TBA(3) | MeHQ(0.01) | DHT(0.008) | D7 (0.5) | | - |
| Example 52 | Bis-GMA(45), UDMA(30), NPG(25) | - | CQ(0.1) | IFB(3) | TBA(3) | MeHQ(0.01) | DHT(0.008) | D8(0.5) | | - |
| Example 53 | UDMA(60), TEGDMA(30) | MHPA(10) | CQ(0.3) BAPO (0.1) | IFB(3) | TBA(3) | MeHQ(0.01) | DHT(0.008) | D7 (0.8) | | - |
| Example 54 | Bis-GMA(45), UDMA(30), NPG(25) | - | BAPO(0.5) | - | DMBE(0.5) | MeHQ(0.01) | DHT(0.01) | D7(0.5) | | E1(320),E3 (80), E6(3) |
| Example 55 | Bis-GMA(60), TEGDMA(40) | - | CQ(0.1) | DPI(0.5) | DMBE(3) | MeHQ(0.01) | DHT(0.01) | D7 (0.1) | | E1(230),E3 (100), E5 (5),E7(6) |

(continued)

| Examples | Matrix | | | | | | | | | (E) filler |
|---|---|---|---|---|---|---|---|---|---|---|
| | (A) Polymerizable monomer | | (B) Polymerization initiator | | | Polymerization inhibitor | (C) Fluorescent agent | (D) Ultraviolet absorber | | |
| | Polymerizable monomer other than (A1) | (A1) Polymerizable monomer having an acidic group | (B-1) Photosensitizer | (B-2) Photoacid generator | (B-3) Polymerization accelerator | | | | | |
| Example 56 | Bis-GMA(45), UDMA(30), NPG(25) | - | CQ(0.2) | IFP(2) | TBA(3) | MeHQ(0.01) | DHT(0.01) | D7 (0.2)<br>D8 (0.2) | | E1(230),E3 (100), E5 (5),E7(6) |
| Comparative Example 1 | Bis-GMA(60), TEGDMA(40) | - | CQ(0.1) | IFB(3) | TBA(3) | MeHQ(0.01) | DHT(0.01) | D3(0.5) | | E1(230),E3 (100), E5 (5),E7(6) |
| Comparative Example 2 | Bis-GMA(60), TEGDMA(40) | - | CQ(0.1) | IFB(3) | TBA(3) | MeHQ(0.01) | DHT(0.01) | D1(0.5) | | E1(230),E3 (100), E5 (5),E7(6) |
| Comparative Example 3 | Bis-GMA(45), UDMA(30), NPG(25) | - | CQ(0.1) | IFB(3) | TBA(3) | MeHQ(0.01) | DHT(0.01) | D4(0.5) | | E1(320),E3 (80), E6(3) |
| Comparative Example 4 | Bis-GMA(45), UDMA(30), NPG(25) | - | CQ(0.1) | IFB(3) | TBA(3) | MeHQ(0.01) | DHT(0.01) | D5(0.5) | | E1(320),E3 (80), E6(3) |
| Comparative Example 5 | Bis-GMA(45), UDMA(30), NPG(25) | - | CQ(0.1) | IFB(3) | TBA(3) | MeHQ(0.01) | DHT(0.01) | D2(0.25)<br>D3(0.25) | | E1(320),E3 (80), E6(3) |
| Comparative Example 6 | UDMA(60), TEGDMA(30) | MHPA(10) | CQ(0.1) | IFB(3) | TBA(3) | MeHQ(0.01) | DHT(0.01) | D6(0.01) | | E2(180),E3 (50), E5(3), E7(5) |
| Comparative Example 7 | UDMA(60), TEGDMA(30) | MHPA(10) | CQ(0.1) | IFB(3) | TBA(3) | MeHQ(0.01) | DHT(0.01) | D6(0.1) | | E2(180),E3 (50), E5(3), E7(5) |
| Comparative Example 8 | UDMA(50), NPG(35) | MDP(15) | CQ(0.1) | IFB(3) | TBA(3) | MeHQ(0.01) | DHT(0.01) | D6(0.5) | | E2(180),E3 (50), E5(5) |

(continued)

| Examples | Matrix | | | | | | | | | (E) filler |
|---|---|---|---|---|---|---|---|---|---|---|
| | (A) Polymerizable monomer | | (B) Polymerization initiator | | | Polymerization inhibitor | (C) Fluorescent agent | (D) Ultraviolet absorber | | |
| | Polymerizable monomer other than (A1) | (A1) Polymerizable monomer having an acidic group | (B-1) Photosensitizer | (B-2) Photoacid generator | (B-3) Polymerization accelerator | | | | | |
| Comparative Example 9 | UDMA(50), NPG(35) | MDP(15) | CQ(0.1) | IFB(3) | TBA(3) | MeHQ(0.01) | DHT(0.01) | D6(1.5) | | E2(180),E3 (50), E5(5) |
| Comparative Example 10 | UDMA(60), TEGDMA(30) | MHPA(10) | CQ(0.1) | IFB(3) | TBA(3) | MeHQ(0.01) | DHT(0.01) | D6(2.0) | | E2(180),E3 (50), E5(3), E7(5) |
| Comparative Example 11 | Bis-GMA(45), UDMA(30), NPG(25) | - | CQ(0.1) | IFB(3) | TBA(3) | MeHQ(0.01) | DHT(0.01) | D1(0.25) D3(0.25) | | E1(320),E3 (80), E6(3) |
| Comparative Example 12 | Bis-GMA(60), TEGDMA(40) | - | CQ(0.1) | IFG(3) | TBA(3) | MeHQ(0.01) | - | D7 (0.1) | | E1(230),E3 (100), E5 (5),E7(6) |

[Table 3]

| Examples | | (A) Polymerizable monomer | | (B) Polymerization initiator | | | | Polymerization inhibitor | (C) Fluorescent agent | (D) Ultraviolet absorber | (E) filler |
|---|---|---|---|---|---|---|---|---|---|---|---|
| | | Polymerizable monomer other than (A1) | (A1) Polymerizable monomer | (8-1) Photosensitizer | (B-2) Photoacid generator | (B-3) Polymerization accelerator | Polymerization inhibitor | | | | |
| Exampte 101 | First paste | Bis-GMA(60), TEGDMA(40) | - | CQ(0.1) | - | TBA(3) | DEPT(1.2) | BHT(0.01) | DHT(0.02) | D7 (0.9) | E1 (200), E3(50), E5(5), E7(6) |
| | Second paste | Bis-GMA(60), TEGDMA(40) | - | - | IFB(3) | - | BPO(1.5) | BHT(0.1) | - | - | E1 (200), E3(50), E5(5), E7(6) |
| Exampte 102 | First paste | Bis-GMA(60), TEGDMA(40) | - | - | IFG(2) | TBA(1.5) | DEPT(1.2) | - | DHT(0.02) | D7 (4) | E1 (200), E3(50), E5(5), E7(6) |
| | Second paste | Bis-GMA(60), TEGDMA(40) | - | CQ(0.2) | - | - | BPO(1.5) | BHT(0.1) | - | - | E1 (200), E3(50), E5(5), E7(6) |
| Exampte 103 | First paste | Bis-GMA(40), TEGDMA(40), UDMA(20) | - | - | - | TBA(1) | DEPT(1.2) | MeHQ(0.01) | DHT(0.02) | D7 (0.9) | E1 (200), E3(50), E5(5), E7(6) |
| | Second paste | Bis-GMA(40), TEGDMA(40), UDMA(20) | - | CQ(0.1) | IFP(1.1) | - | BPO(1.5) | BHT(0.1) | - | - | E1 (200), E3(50), E5(5), E7(6) |

(continued)

| Examples | | (A) Polymerizable monomer | | (B) Polymerization initiator | | | | Polymerization inhibitor | (C) Fluorescent agent | (D) Ultraviolet absorber | (E) filler |
|---|---|---|---|---|---|---|---|---|---|---|---|
| | | Polymerizable monomer other than (A1) | (A1) Polymerizable monomer | (8-1) Photosensitizer | (B-2) Photoacid generator | (B-3) Polymerization accelerator | Polymerization inhibitor | | | | |
| Exampte 104 | First paste | Bis-GMA(60), TEGDMA(40) | MDP(20) | CQ(0.1) | - | - | TMBH(3) | MeHQ(0.01) | DHT(0.02) | D7 (0.9) | E2 (200), E5(6) |
| | Second paste | Bis-GMA(60), TEGDMA(40) | - | - | IFB(3) | TBA(3) | PTU(3) | BHT(0.1) | - | - | E1 (150), E3 (50), E5(5) |
| Exampte 105 | First paste | UDMA(50), TEGDMA(40) | MHPA(10) | CQ(0.1) | - | TBA(3) | TMBH(3) | BHT(0.1) | DHT(0.02) | D7 (0.5) | E2 (230), E6(10) |
| | Second paste | UDMA(60), TEGDMA(40) | - | - | IFB(2) | - | PTU(3), VOA (0.005) | BHT(0.1) | - | - | E1 (150), E3(50), E6(10) |
| Exampte 106 | First paste | UDMA(50), TEGDMA(20), HEMA(20) | MDP(10) | CQ(0.1) | - | TBA(3) | TMBH(3) | BHT(0.1) | DHT(0.02) | D7 (0.5) | E2 (230), E6(10) |
| | Second paste | UDMA(60), TEGDMA(40) | - | - | IFB(2) | - | PTU(3), CAA (0.05) | BHT(0.1) | - | - | E1 (150), E3(50), E6(10) |
| Exampte 107 | First paste | UDMA(60), TEGDMA(40) | MDP(10) | CQ(0.2) | - | TBA(2) | TMBH(3) | BHT(0.1) | DHT(0.02) | D7 (0.5) | E2 (230), E6(10) |
| | Second paste | UDMA(60), TEGDMA(40) | - | - | IFB(5) | - | PTU(3),CAA (0.1), KPS(0,5) | BHT(0.1) | - | - | E1 (150), E3(50), E6(10) |

(continued)

| Examples | | (A) Polymerizable monomer | | (B) Polymerization initiator | | | | Polymerization inhibitor | (C) Fluorescent agent | (D) Ultraviolet absorber | (E) filler |
|---|---|---|---|---|---|---|---|---|---|---|---|
| | | Polymerizable monomer other than (A1) | (A1) Polymerizable monomer | (8-1) Photosensitizer | (B-2) Photoacid generator | (B-3) Polymerization accelerator | Polymerization inhibitor | | | | |
| Exampte 108 | First paste | UDMA(50), TEGDMA(20), HEMA(20) | MDP(10) | CQ(0.2) | - | TBA(2) | PTU(3) | - | DHT(0.02) | D7 (0.5) | E2 (230), E6(10) |
| | Second paste | UDMA(60), TEGDMA(40) | - | - | IFB(3) | - | TMBH(3) | BHT(0.1) | - | - | E1 (150), E3(50), E6(10) |
| Exampte 109 | First paste | UDMA(50), TEGDMA(20), HEMA(20) | MDP(10) | CQ(0.2) | - | - | TMBH(3) | - | DHT(0.02) | D7 (0.5) | E2 (230), E6(10) |
| | Second paste | UDMA(60), TEGDMA(40) | - | - | - | TBA(2) | PTU(2), CAA (0.1) | BHT(0.1) | - | - | E1 (150), E3(50), E6(10) |
| Exampte 110 | First paste | UDMA(50), TEGDMA(20), HEMA(20) | MDP(10) | - | - | - | PTU(2), VOA (0.05) | - | DHT(0.02) | D7 (0.5) | E2 (230), E6(10) |
| | Second paste | UDMA(60), TEGDMA(40) | - | - | - | - | TMBH(4) | BHT(0.1) | - | - | E1 (150), E3(50), E6(10) |
| Comparative Exampte 101 | First paste | Bis-GMA(60), TEGDMA(40) | - | CQ(0.1) | - | - | TMBH(3) | MeHQ(0.01) | DHT(0.02) | D4 (1) | E1 (250), E3(80), E6(3) |
| | Second paste | Bis-GMA(60), TEGDMA(40) | - | - | IFB(3) | TBA(3) | PTU(3) | BHT(0.1) | - | - | E1 (250), E3(80), E6(3) |

(continued)

| Examples | | (A) Polymerizable monomer | | (B) Polymerization initiator | | | | Polymerization inhibitor | (C) Fluorescent agent | (D) Ultraviolet absorber | (E) filler |
|---|---|---|---|---|---|---|---|---|---|---|---|
| | | Polymerizable monomer other than (A1) | (A1) Polymerizable monomer | (8-1) Photosensitizer | (B-2) Photoacid generator | (B-3) Polymerization accelerator | Polymerization inhibitor | | | | |
| Comparative Exampte 102 | First paste | UDMA(50), TEGDMA(20), HEMA(20) | MDP(10) | CQ(0.2) | - | - | PTU(2), CAA (0.1) | - | DHT(0.02) | D1(0.5) | E2 (230), E6(10) |
| | Second paste | UDMA(60), TEGDMA(40) | - | - | IFB(3) | - | TMBH(3) | BHT(0.1) | - | D3(0.5) | E1 (150), E3(50), E6(10) |

(1) Fluorescence

Prepared dental curable composition was fully filled into a mold (in a shape of a disc having a diameter of 15 mm and a thickness of 1 mm) made of stainless steel. Thereafter, a cover glass was placed on upper side of the stainless mold to apply pressure with glass plate. Then, light irradiation was performed for 1 minute using a photopolymerization irradiator (PEN Bright manufactured by SHOFU INC.) via the cover glass to prepare a cured material. After removing the cover glass, the cured material was taken out of the mold. This cured material was used as a test specimen for evaluating fluorescence. The test specimen and a natural tooth were irradiated with ultraviolet light with a wavelength of 366 nm, and the fluorescence state of each was observed and evaluated by three evaluators. Evaluation criteria were as follows and the most common evaluation was used.

A (Good): fluorescence was equivalent to that of a natural tooth
B (Applicable): fluorescence was slightly poor or wass slightly stronger than that of a natural tooth, but there are no problems in use.
C (Unsuitable): fluorescence was poor or was strong compared to a natural tooth, and there is a problem in use.
D (Unsuitable): fluorescence was clearly poor or was clearly strong compared to a natural tooth, therefore it was unnatural and there is a problem in use.

When fluorescence is too strong or too weak compared to a natural tooth, in the case of viewing under light source containing ultraviolet light such as sunlight, a repaired portion is conspicuous because of emitting light differently from a natural tooth, and therefore is not aesthetically preferable.

(2) Flexural strength

The prepared dental curable composition was filled into a stainless steel mold, and the cover glasses were placed on both sides to press with a glass kneading plate. Thereafter, light was irradiated for 10 seconds to 5 locations by using the photopolymerization irradiator (PEN Bright manufactured by SHOFU INC.) to cure the dental photocurable composition. After curing, the cured product was removed from the mold, and light was irradiated to the backside in the same manner again to use as a test specimen (25 × 2 × 2 mm rectangular shape). The test specimen was immersed in water at 37 °C for 24 hours, and thereafter flexural test was performed. The flexural test was conducted at a distance between supporting points of 20 mm and at a crosshead speed of 1 mm/min using an Instron universal testing machine (manufactured by Instron). Evaluation criteria were as follows.

Particularly good: 120 MPa or more
Good: 100 or more and less than 120 MPa
Insufficient: less than 100 Mpa

(3) Sunlight stability

Prepared dental curable composition was fully filled into a mold (in a shape of a disc having a diameter of 15 mm and a thickness of 1 mm) made of stainless steel. Thereafter, a cover glass was placed on upper side of the stainless mold to apply pressure with glass plate. Then, light irradiation was performed for 1 minute using a photopolymerization irradiator (PEN Bright manufactured by SHOFU INC.) via the cover glass to prepare a cured material. After removing the cover glass, the cured material was taken out of the mold. The color tone of the cured material was measured. Color measurement was performed by placing the test specimen on the background of a standard white plate (D65/10°, X=81.07, Y=86.15, Z=93.38) and using a spectrocolorimeter (manufactured by BYK-Chemie GmbH) under predetermined condition (light source: C, viewing angle: 2°, measurement area: 11 mm). Then, after exposing the test specimen to light for 24 hours with a xenon lamp light exposure tester (Suntest CPS +), the color tone of the test specimen was measured again, and the difference in discoloration was represented by ΔE calculated from the following formula.

$$\Delta E = ((\Delta L^*)^2 + (\Delta a^*)^2 + (\Delta b^*)^2)^{1/2}$$

$$\Delta L^* = L1^* - L2^*$$

$$\Delta a^* = a1^* - a2^*$$

$$\Delta b^* = b1^* - b2^*$$

In the formula, L1* is the brightness index before light exposure, L2* is the brightness index after light exposure, a1* and b1* are the color quality index before light exposure, and a2* and b2* are the color quality index after light exposure. Evaluation criteria were as follows.

A (Good): ΔE was less than 3
B (Applicable): ΔE was less than 5
C (Poor): ΔE was 5 or more

The Sunlight stability was measured for predict the color tone change when the cured product was used for a long period of time in the light exposed area, and the smaller Δ E, the smaller the color change when the cured product was exposed with light for a long period of time. The dental curable composition that exhibits little change in color can be suitably used as a dental material for which aesthetic property is important.

(4) Solubility

When the matrix was prepared, all ingredients except for the (E) filler and chemical polymerization initiator of each Example were put into a wide mouthed plastic container and mixed by using a stirrer (mix rotor VMRC-5 manufactured by AS ONE) set to 50 °C.
When the matrix was prepared, all ingredients except for the (E) filler and chemical polymerization initiator of each Example were put into a wide mouthed plastic container and mixed by using a stirrer (mix rotor VMRC-5 manufactured by AS ONE) set to 50 °C. After mixing has started, visual inspection was performed to see whether any solids remain at 3 hours, 36 hours, and 48 hours.
Evaluation criteria were as follows.

A (Excellent): No solid matter remained after 3 hours.
B (Good): Solid matter was present after 3 hours, but no solid matter was present after 36 hours.
C (Unsuitable): Solid matter was present after 36 hours, but no solid matter was present after 48 hours.

Good solubility is preferable because it allows for short production times. Furthermore, it is preferable because there may be a low risk of the photo acid generator precipitating during storage at low temperatures. On the other hand, an ultraviolet absorbent having poor solubility is not preferable because there is a case of precipitant after long-term storage even if it is uniformly dissolved in the composition. Furthermore, it takes a long time to produce the composition, and it is necessary to expose the composition to high temperatures for a long period of time, therefore there is a case where deterioration of the materials during preparation is caused, thus it is not preferable.
The results shown in Tables 4 and 5 will be described.

[Table 4]

| Examples | Fluorescence | Flexural strength | Sunlight stability | Solubility | Examples | Fluorescence | Flexural strength | Sunlight stability | Solubility |
|---|---|---|---|---|---|---|---|---|---|
| | | MPa | ⊿E | | | | MPa | ⊿E | |
| Example 1 | A | 127 | 4.8 | A | Example 36 | B | 118 | 2.3 | A |
| Example 2 | A | 123 | 4.2 | A | Example 37 | B | 104 | 2 | A |
| Example 3 | A | 129 | 3.7 | A | Example 38 | A | 125 | 4.9 | B |
| Example 4 | A | 129 | 2.7 | A | Example 39 | A | 120 | 4.3 | B |
| Example 5 | A | 126 | 2.8 | A | Example 40 | A | 121 | 2.8 | B |
| Example 6 | A | 123 | 2.7 | A | Example 41 | A | 123 | 2.9 | B |
| Example 7 | A | 121 | 2.9 | A | Example 42 | A | 123 | 4.9 | B |
| Example 8 | A | 112 | 1.1 | A | Example 43 | A | 116 | 2.3 | B |
| Example 9 | A | 102 | 1.3 | A | Example 44 | A | 110 | 2.3 | B |
| Example 10 | A | 104 | 1.1 | A | Example 45 | A | 112 | 4.2 | B |
| Example 11 | A | 111 | 1.4 | A | Example 46 | A | 114 | 2.3 | B |
| Example 12 | A | 122 | 1.8 | A | Example 47 | A | 104 | 2.1 | B |
| Example 13 | A | 121 | 2.6 | A | Example 48 | B | 115 | 4.5 | B |
| Example 14 | A | 112 | 3.2 | A | Example 49 | A | 115 | 2.1 | B |
| Example 15 | A | 116 | 4.1 | A | Example 50 | B | 122 | 1.9 | B |
| Example 16 | A | 124 | 4.5 | A | Example 51 | A | 113 | 2.4 | A |
| Example 17 | A | 123 | 4.7 | A | Example 52 | A | 115 | 2.3 | B |
| Example 18 | A | 124 | 4.8 | A | Example 53 | A | 114 | 1.8 | A |
| Example 19 | A | 123 | 2.8 | A | Example 54 | A | 113 | 4.2 | A |
| Example 20 | A | 108 | 2.3 | A | Example 55 | A | 124 | 4.9 | A |
| Example 21 | A | 122 | 2.2 | A | Example 56 | A | 121 | 2.5 | B |
| Example 22 | A | 125 | 4.8 | A | Comparative Example 1 | B | 120 | 127 | B |
| Example 23 | A | 110 | 4.8 | A | Comparative Example 2 | A | 124 | 18.8 | B |
| Example 24 | B | 122 | 2.2 | A | Comparative Example 3 | D | 97 | 9.6 | B |

(continued)

| Examples | Fluorescence | Flexural strength | Sunlight stability | Solubility | Examples | Fluorescence | Flexural strength | Sunlight stability | Solubility |
|---|---|---|---|---|---|---|---|---|---|
| | | MPa | ⊿E | | | | MPa | ⊿E | |
| Example 25 | A | 125 | 2.3 | A | Comparative Example 4 | C | 105 | 7.3 | B |
| Example 26 | A | 114 | 1.8 | A | Comparative Example 5 | C | 114 | 8.4 | C |
| Example 27 | B | 118 | 1.5 | A | Comparative Example 6 | C | 113 | 7.8 | B |
| Example 28 | A | 127 | 1.9 | A | Comparative Example 7 | C | 115 | 5.3 | B |
| Example 29 | A | 126 | 1.9 | A | Comparative Example 8 | C | 113 | 4.2 | B |
| Example 30 | A | 121 | 1.9 | A | Comparative Example 9 | D | 108 | 3.0 | B |
| Example 31 | A | 111 | 1.4 | A | Comparative Example 10 | D | 102 | 2.9 | B |
| Example 32 | B | 109 | 1.2 | A | Comparative Example 11 | A | 117 | 10.7 | C |
| Example 33 | B | 124 | 3.1 | A | Comparative Example 12 | D | 125 | 2.6 | A |
| Example 34 | B | 122 | 2.8 | A | | | | | |
| Example 35 | B | 123 | 2.7 | A | | | | | |

[Table 5]

| Examples | | uorescenc | Flexural strength | Sunlight stability | Solubility |
|---|---|---|---|---|---|
| | | | MPa | ⊿E | |
| Example 101 | First paste | A | 122 | 4.4 | A |
| | Second paste | | | | |
| Example 102 | First paste | B | 108 | 2.7 | A |
| | Second paste | | | | |
| Example 103 | First paste | A | 121 | 4.3 | A |
| | Second paste | | | | |
| Example 104 | First paste | A | 124 | 2.6 | A |
| | Second paste | | | | |
| Example 105 | First paste | A | 129 | 2.4 | A |
| | Second paste | | | | |
| Example 106 | First paste | A | 128 | 2.9 | A |
| | Second paste | | | | |
| Example 107 | First paste | A | 129 | 2.8 | A |
| | Second paste | | | | |
| Example 108 | First paste | A | 129 | 2.7 | A |
| | Second paste | | | | |
| Example 109 | First paste | A | 111 | 2.5 | A |
| | Second paste | | | | |
| Example 110 | First paste | A | 101 | 2.6 | A |
| | Second paste | | | | |
| Comparative Example 101 | First paste | D | 95 | 9.4 | B |
| | Second paste | | | | |
| Comparative Example 102 | First paste | A | 115 | 10.1 | C |
| | Second paste | | | | |

It was confirmed that the compositions described in Examples had good fluorescence, flexural strength, sunlight stability, and solubility.

In Examples 9 to 10, because the compounding amount of the polymerization initiator was small, there was a tendency that flexural strength was poor. In Examples 16 to 18 and 42, because the compounding amount of the polymerization initiator was large, there was a tendency that flexural strength was good, but sunlight stability was poor.

In Examples 24, 33, 34 and 48, because the compounding amount of the fluorescent agent was small, there was a tendency that fluorescence was slightly poor. In Examples 27, 37 and 50, because the compounding amount of the fluorescent agent was large, there was a tendency that fluorescence is too high to cause unnatural light emission or physical property is poor.

In Examples 1, 2 and 38, because the compounding amount of the ultraviolet absorber was small, there was a tendency that sunlight stability was poor. In Examples 31, 32 and 47, because the compounding amount of the ultraviolet absorber was large, there was a tendency that sunlight stability was good, but flexural strength was poor.

In Examples 1 to 37, because of containing D7 which is a benzophenone compound represented by formula 1, there was a tendency that solubility was particularly excellent.

In Examples 7, 20 and 44, because of containing TBA and DM which are aliphatic amine, there was a tendency that sunlight stability results were more excellent than Examples 23, 45 and 55 containing DMBE which is aromatic amine.

In Examples 20 and 23, because of not containing the (B-2) photoacid generator, there was a tendency that

flexural strength was poor as compared to Examples 21 and 22 which contained (B-2) photoacid generator.

In Comparative Examples 1 and 2, because of using an ultraviolet absorber having an absorption maximum in only one of the wavelength regions of 250 to 320 nm or 320 to 400 nm, there was a tendency that sunlight stability was significantly poor.

In Comparative Examples 3 to 10, because of using an ultraviolet absorber in which the ratio of an absorbance of the first absorption maximum to an absorbance of the second absorption maximum is less than 1, there was a tendency that fluorescences was significantly poor.

In Comparative Examples 6 to 10, because of using an ultraviolet absorber in which the ratio of an absorbance of the first absorption maximum to an absorbance of the second absorption maximum is less than 1, there was a tendency that the sunlight stability was improved by increasing the compounding amount of the ultraviolet absorber, but fluorescence was significantly poor.

In Comparative Examples 5 to 11, because of using a combination of ultraviolet absorbers having an absorption maximum in only one of the wavelength regions of 250 to 320 nm or 320 to 400 nm, there was a tendency that the solubility was significantly poor.

It was confirmed in Examples 101 to 110 which are examples of two packs type dental curable composition that the compositions had good fluorescence, flexural strength, sunlight stability, and solubility. Among Examples 101 to 103 containing p-tolyldiethanolamine which is an aromatic amine compound, Example 103 in which the compounding amount of the ultraviolet absorber was excessive exhibited the tendency that sunlight stability was good, but flexural strength and fluorescence decreased. On the other hand, in Examples 101 and 103, there was a tendency that sunlight stability was poor as compared to Examples 104 to 110.

Comparative Examples 101 and 102 are comparative Examples of two packs type dental curable composition. In Comparative Examples 101 containing D4 which is an ultraviolet absorber in which the ratio of an absorbance of the first absorption maximum to an absorbance of the second absorption maximum is less than 1, there was a tendency that the sunlight stability was improved by increasing the compounding amount of the ultraviolet absorber, but fluorescence was significantly poor. In Comparative Examples 102 containing a combination of D1 and D3 which are an ultraviolet absorber in which the ratio of an absorbance of the first absorption maximum to an absorbance of the second absorption maximum is more than 4.0, there was a tendency that the sunlight stability was good, but sunlight stability was poor and solubility of an ultraviolet absorber with respect to the matrix was poor.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this disclosure without difficulty. Accordingly, any such changes and modifications are intended to be included in the scope disclosure the embodiments.

Industrial applicability

The present invention can provide a dental curable composition having excellent solubility of an ultraviolet absorber, sunlight stability, fluorescence, and mechanical property.

## Claims

**1.** A dental curable composition comprising,

(A) polymerizable monomer, (B) polymerization initiator, (C) fluorescent agent, and (D) ultraviolet absorber, wherein,

the dental curable composition comprises, as the (D) ultraviolet absorber, (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the range of 1 to 4.

**2.** The dental curable composition according to claim 1, wherein the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the range of 1 to 4 is a benzophenone compound represented by formula (1).

[Formula (1)]

[Chemical formula 1]

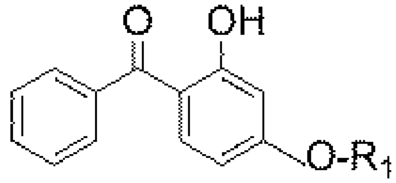

(In the formula (1), $R_1$ is an organic group having 2 to 10 carbon atoms.)

3. The dental curable composition according to claim 1 or 2, wherein

   the dental curable composition comprises a combination of (B-1) photosensitizer, (B-2) photoacid generator and (B-3) polymerization accelerator as the (B) polymerization initiator,
   the dental curable composition comprises a diaryl iodonium salt compound as the (B-2) photoacid generator, and
   the dental curable composition comprises a tertiary amine compound as the (B-3) polymerization accelerator.

4. The dental curable composition according to any one of claims 1 to 3, wherein the dental curable composition does not substantially comprise an aromatic amine compound as the (B-3) polymerization accelerator.

5. The dental curable composition according to any one of claims 1 to 4, wherein the dental curable composition comprises only the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4 as the (D) ultraviolet absorber.

6. The dental curable composition according to any one of claims 1 to 5, wherein the dental curable composition comprises a terephthalic acid ester compound represented by formula (3) as the (C) fluorescent agent.

[Formula (3)]

[Chemical formula 2]

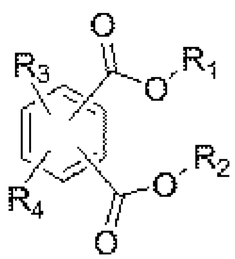

(In the formula, $R_1$ and $R_2$ each independently represent an alkyl group, $R_3$ represents a hydrogen atom, an amino group or a hydroxyl group, and $R_4$ represents an amino group or a hydroxyl group. The alkyl group has 1 to 3 carbon atoms, such as a methyl group, an ethyl group, an n-propyl group, or an i-propyl group.)

7. The dental curable composition according to any one of claims 1 to 6, wherein the dental curable composition comprises only a terephthalic acid ester compound represented by formula (3) as the (C) fluorescent agent.

[Formula (3)]

[Chemical formula 3]

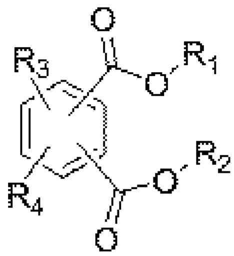

(In the formula, $R_1$ and $R_2$ each independently represent an alkyl group, $R_3$ represents a hydrogen atom, an amino group or a hydroxyl group, and $R_4$ represents an amino group or a hydroxyl group. The alkyl group has 1 to 3 carbon atoms, such as a methyl group, an ethyl group, an n-propyl group, or an i-propyl group.)

**8.** The dental curable composition according to any one of claims 1 to 7, wherein the dental curable composition comprises, with respect to 100 parts by mass of the (A) polymerizable monomer,

0.5 to 10 parts by mass of the (B) polymerization initiator,
0.001 to 0.1 parts by mass of the(C) fluorescent agent, and
0.01 to 2 parts by mass of the (D-1) compound having at least one first absorption maximum in the wavelength region of 250 to 320 nm and at least one second absorption maximum in the wavelength region of 320 to 400 nm, wherein the ratio of an absorbance of the first absorption maximum to an absorbance of the second absorption maximum is within the tange of 1 to 4.

Europäisches Patentamt
European Patent Office
Office européen des brevets

# EUROPEAN SEARCH REPORT

Application Number
EP 24 17 9491

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2023 042484 A (SHOFU INC)<br>27 March 2023 (2023-03-27)<br>* claim 1 *<br>* paragraphs [0136] - [0159] *<br>* table 2 *<br>----- | 1-8 | INV.<br>A61K6/16<br>A61K6/20<br>A61K6/30<br>A61K6/60<br>A61K6/62<br>A61K6/887 |
| | | | TECHNICAL FIELDS SEARCHED (IPC) |
| | | | A61K |

The present search report has been drawn up for all claims

1

| Place of search | Date of completion of the search | Examiner |
|---|---|---|
| The Hague | 1 November 2024 | Gomes Pinto F., R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 17 9491

This annex lists the patent family members relating to the patent documents cited in the above-mentioned European search report. The members are as contained in the European Patent Office EDP file on
The European Patent Office is in no way liable for these particulars which are merely given for the purpose of information.

01-11-2024

| Patent document cited in search report | Publication date | Patent family member(s) | Publication date |
|---|---|---|---|
| JP 2023042484 A | 27-03-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004231913 A
- JP 2014015408 A
- JP 2016166138 A
- WO 2020066099 A